# DEMANDE DE BREVET EUROPEEN

(11) **EP 4 303 318 A1**
(43) Date de publication de la demande: **10.01.2024**
(21) Numéro de dépôt: 22183391.6
(22) Date de dépôt: 06.07.2022
(51) Int. Cl.: C12Q 1/6883, C12Q 1/70

(54) **DETERMINATION DU RISQUE DE DECES D'UN SUJET INFECTE PAR UN VIRUS RESPIRATOIRE PAR LA MESURE DU NIVEAU D EXPRESSION DU GENE ADGRE3**

(71) Demandeur: BIOMÉRIEUX, 69280 Marcy L'Etoile (FR); Hospices Civils de Lyon, 69002 Lyon (FR); UNIVERSITE CLAUDE BERNARD - LYON 1, 69100 Villeurbanne (FR)
(72) Inventeur: TARDIVEAU, Claire, 69650 Saint Germain au Mont d'Or (FR); LUKASZEWICZ, Anne-Claire, 69006 LYON (FR); MONNERET, Guillaume, 69003 LYON (FR); VENET, Fabienne, 69003 LYON (FR)
(74) Mandataire: bioMérieux PI Groupement mandataires

(57) **Abrégé**

L'invention concerne un procédé de détermination *in vitro* ou *ex vivo* pour déterminer le risque de décès chez un sujet infecté par un virus respiratoire, ledit procédé comprenant les étapes de mesure dans un échantillon biologique dudit sujet du niveau d'expression du gène ADGRE3, et de comparaison du niveau d'expression ainsi mesurée ou une valeur dérivée de cette quantité, à une valeur de référence prédéterminée. Le procédé permet ainsi de conclure à un risque accru de décès du sujet dès lors qu'une sous-expression du gène ADGRE3 est statistiquement mise en évidence à partir de l'échantillon biologique. De manière avantageuse, la mesure du niveau d'expression de ADGRE3 peut être complétée par celle d'un ou plusieurs gènes additionnels tels que C3AR1, CD177, OAS2, CIITA, IL-10, IL1R2, CD74, TDRD9 et leurs combinaisons. L'invention concerne également des kits permettant la mesure de l'expression de ADGRE3 et éventuellement d'un ou plusieurs gènes additionnels.

## Description

### Domaine technique

La présente invention concerne le domaine technique des procédés et kits de diagnostic *in vitro.* En particulier, l'invention a pour objet des procédés et kits permettant de déterminer la présence d'un risque accru de décès chez un sujet infecté par un virus respiratoire, notamment par un virus respiratoire tel que le SARS-CoV-2 ou l'un de ses variants.

### Technique antérieure

La pandémie de la maladie liée au coronavirus (COVID-19) causée par le coronavirus 2 du syndrome respiratoire aigu sévère (SARS-CoV-2) a infecté à ce jour plus de 550 millions de patients dans le monde et provoqué plus de 6,3 millions de décès.

La gravité de la maladie varie considérablement d'un patient à l'autre et la majorité des patients est asymptomatique ou présente des symptômes minimes tels que la fièvre, la toux et/ou un essoufflement. Néanmoins, 5 à 10 % des patients nécessitent des soins intensifs en raison de l'évolution rapide (9 à 12 jours) vers une forme grave ou critique avec le développement, par exemple, de syndrome de détresse respiratoire aiguë (SDRA) et/ou d'hypoxémie sévère, de lésions pulmonaires aiguës (LPA), de défaillance de plusieurs organes, voire de décès (C. Huang *et al.,* 2020).

La réponse immunitaire joue un rôle prépondérant dans la physiopathologie de la COVID-19 et le phénotype le plus sévère lors de l'admission en soins intensifs des patients infectés se caractérise par un profil immunitaire complexe qui évolue au fil du temps (E.Z. Ong *et al.,* 2020).

Malgré tout, chez les patients sévères atteints de la COVID-19, la réponse immunitaire peut être définie par une altération des réponses inflammatoires et immunitaires avec une lymphopénie marquée, un nombre élevé de neutrophiles et de monocytes, une diminution de l'expression HLA-DR des monocytes, une tempête modérée de cytokines plasmatiques, une réponse inadéquate de la signalisation des interférons de type I et une régulation négative des gènes stimulés par l'interféron (ISG) (F. Venet et al., Crit Care, 2021). Ces altérations peuvent conduire à une microthrombose et à des lésions tissulaires, entraînant finalement un SDRA, une défaillance de plusieurs organes et la mort (Hadjadj J *et al.,* 2020).

Pendant la pandémie, de nombreuses études exploratoires ont été menées pour déchiffrer les processus immunitaires impliqués dans la COVID-19. Dans l'ensemble, ces études ont utilisé diverses approches mixtes de flux (flux spectral, flux multicolore, spectrométrie de masse à temps de vol), des tests fonctionnels, mais également la mesure multiplex des médiateurs solubles. Les résultats ont été principalement analysés par des approches multi-données/-omiques. Bien qu'elles fournissent des informations cruciales sur la physiopathologie du COVID-19, ces approches sont principalement basées sur des outils de recherche clinique qui ne sont pas utilisables en routine clinique au chevet des patients ou au laboratoire central pour caractériser le profil immunitaire et donc les éventuels risques de décès, et ce, en raison d'un nombre important de limitations : temps de mise en œuvre conséquent, manque de standardisation, faible reproductivité entre cohortes, coût substantiel.

Par conséquent, il existe un besoin de développer des approches alternatives pour permettre dans un temps réduit et au chevet du patient ou en laboratoire central, de prédire ou identifier efficacement le risque de mortalité des patients infectés par des virus respiratoires tels que ceux responsables de la COVID-19. En ce sens, la mesure de biomarqueur(s), notamment transcriptomique(s), est une piste en constante exploration.

Dans ce contexte, il a notamment été établi que les trajectoires longitudinales de 11 biomarqueurs circulants basés sur l'immunité pouvaient être associées à la mortalité des patients lorsqu'elles étaient augmentées (10) ou diminuées (1), fournissant ainsi un début de preuve que des biomarqueurs basés sur l'immunité pourraient permettre d'obtenir une alerte précoce de l'issue des patients atteints par la COVID-19 (Abers *et al.,* 2021).

Des profils d'expression géniques ont également été décrits pour prédire l'issue des patients atteints de la COVID-19 (Guardela B *et al.,* 2021).

C'est ainsi que le biomarqueur CD177 a été décrit comme pouvant être associé à la sévérité et au risque de décès des patients atteints du COVID-19. Particulièrement, la stabilité des niveaux protéiques de CD177 dans le sérum chez les patients atteints de COVID-19 sévère au cours de la maladie est décrit comme le signe d'un pronostic plus défavorable, pouvant conduire au décès (Levy Y *et al.,* 2021).

Néanmoins, et au regard de la prévalence mondiale, il est toujours nécessaire d'identifier de nouveaux biomarqueurs afin de compléter l'arsenal du clinicien avec d'autres alternatives permettant de prédire ou d'identifier efficacement le risque de décès des sujets infectés par des virus respiratoires, notamment ceux responsables de la COVID-19, afin de pouvoir adapter la prise en charge, préférentiellement de manière précoce, au travers de thérapies guidées et d'améliorer ainsi leurs chances de survie.

### Résumé

Un premier objet de l'invention concerne un procédé *in vitro* ou *ex vivo* pour déterminer le risque de décès chez un sujet infecté par un virus respiratoire, ledit procédé comprenant une étape de mesure dans un échantillon biologique dudit sujet du niveau d'expression du gène ADGRE3, suivie d'une étape de comparaison du niveau d'expression du gène ADGRE3 ainsi mesuré, notamment au niveau ARNm, ou une valeur dérivée dudit niveau, à une valeur de référence prédéterminée.

Sur la base du résultat de la comparaison, une conclusion quant au risque de décès accru chez ledit sujet est émise dès lors qu'une diminution de l'expression du gène ADGRE3 est identifiée.

De manière avantageuse, la valeur de référence correspond à la moyenne du niveau d'expression de ADGRE3 obtenu à partir d'échantillons biologiques issus d'une population de sujets ne présentant aucune infection par un virus respiratoire ou à la moyenne du niveau d'expression de ADGRE3 obtenue à partir d'échantillons biologiques issus d'une population de sujets infectés par un virus respiratoire et dont on sait qu'ils ont survécus après l'infection, notamment dans les 28 jours suivants l'admission en établissement de santé ou dans les 37 jours post infection.

Dans un mode de réalisation préféré, le virus respiratoire est le SARS-CoV-2 ou l'un de ses variants.

De manière avantageuse, la performance de la détermination du risque de décès peut être améliorée par la mesure du niveau d'expression de gènes additionnels. Ainsi, selon un mode de réalisation particulier, le procédé comprend également une étape de mesure dans l'échantillon biologique dudit sujet du niveau d'expression d'au moins un gène additionnel choisi parmi C3AR1, CD177, OAS2, CIITA, IL-10, IL1R2, CD74, TDRD9 et leurs combinaisons, de préférence choisi parmi C3AR1, CD177, OAS2, CIITA, IL-10, IL1R2 et leurs combinaisons, et de préférence encore, choisi parmi C3AR1, OAS2, CIITA, IL-10 et leurs combinaisons.

L'expression mesurée du ou des gènes additionnels et ensuite comparée à une valeur de référence du niveau d'expression respectif desdits gènes et il pourra être conclu à un risque accru de décès chez ledit sujet lorsque la comparaison du niveau d'expression du gène ADGRE3, notamment au niveau ARNm, avec une valeur seuil prédéterminée montre qu'il y a une diminution du niveau d'expression ou une sous expression, et lorsque la comparaison du niveau de transcrits ARNm des gènes additionnels ci-après avec une valeur de référence de leur niveau d'expression respectif, montre qu'il y a :
- une augmentation du niveau d'expression ou une surexpression de C3AR1, et/ou
- une augmentation du niveau d'expression ou une surexpression de CD177, et/ou
- une augmentation du niveau d'expression ou une surexpression de OAS2, et/ou
- une diminution du niveau d'expression ou une sous expression de CIITA, et/ou
- une augmentation du niveau d'expression ou une surexpression de IL-10, et/ou
- une augmentation du niveau d'expression ou une surexpression de IL1R2, et/ou
- une diminution du niveau d'expression ou une sous expression de CD74, et/ou
- une augmentation du niveau d'expression ou une surexpression de TDRD9.

Selon un mode de réalisation préféré, l'échantillon biologique est un échantillon de sang, de préférence un échantillon de sang total.

Selon un autre mode de réalisation préféré, l'expression de ADGRE3, et éventuellement celle des gènes additionnels, est mesurée au niveau ARN messager (ARNm).

Selon un mode de réalisation préféré, l'expression est mesurée par une méthode de détection moléculaire, telle par exemple que l'amplification, le séquençage ou l'hybridation. De préférence, l'expression est mesurée par amplification via une RT-PCR, notamment une RT-qPCR.

Selon un mode de réalisation préféré, l'expression mesurée de ADGRE3, et éventuellement celle des gènes additionnels, est normalisée par rapport à l'expression d'un ou plusieurs gènes de ménage. De préférence, l'expression est normalisée par rapport aux gènes de ménage choisis parmi DECR1, HPRT1, PPIB, GAPDH, ACTB et leurs combinaisons.

Un autre objet de l'invention concerne un kit pour la mesure in vitro ou ex vivo de l'expression de ADGRE3 dans un échantillon biologique comprenant des moyens de détermination du niveau d'expression de ADGRE3 dans ledit échantillon. De préférence, les moyens de détermination sont choisis parmi des amorces d'amplification ou des sondes.

Avantageusement, le kit peut comprendre un échantillon contrôle positif calibré pour contenir la quantité de ADGRE3 qui correspond à la quantité ou la concentration représentative du niveau d'expression mesuré dans un pool d'échantillons de sujets ne présentant pas d'infection par un virus respiratoire ou de sujets infectés par un virus respiratoire et dont on sait qu'ils ont survécu, et/ou un échantillon contrôle négatif calibré pour contenir la quantité de ADGRE3 qui correspond à la quantité moyenne mesurée dans un pool d'échantillons de sujets qui n'ont pas survécu suite à une infection par un virus respiratoire.

Le kit selon l'invention peut également comprendre des moyens de détermination du niveau d'expression d'au moins un gène additionnel sélectionné parmi C3AR1, CD177, OAS2, CIITA, IL-10, IL1R2, CD74, TDRD9 et leurs combinaisons, de préférence sélectionné parmi C3AR1, CD177, OAS2, CIITA, IL-10, IL1R2 et leurs combinaisons, et de préférence encore sélectionné parmi C3AR1, OAS2, CIITA, IL-10 et leurs combinaisons. De préférence, lesdits moyens de détermination sont choisis parmi des amorces d'amplification ou des sondes.

Enfin, un autre objet de l'invention concerne l'utilisation du kit selon l'invention pour la détermination du risque de décès, de préférence du risque de décès dans les 28 jours suivant l'admission en établissement de santé ou dans les 37 jours post infection, d'un sujet infecté par un virus respiratoire tel que le SARS-CoV-2 ou l'un de ses variants.

### Description des modes de réalisation

Certains termes et expressions utilisés dans le cadre de l'invention sont détaillés ci-après.

Un premier objet de l'invention concerne un procédé *in vitro* ou *ex vivo* pour déterminer le risque de décès chez un sujet infecté par un virus respiratoire, ledit procédé comprenant les étapes suivantes de :
a) mesure dans un échantillon biologique dudit sujet du niveau d'expression du gène ADGRE3,
b) comparaison du niveau d'expression du gène ADGRE3 mesuré à l'étape a) ou une valeur dérivée de cette quantité, à une valeur de référence prédéterminée.

D'une manière tout à fait surprenante, il a été constaté que la mesure de l'expression du gène ADGRE3 permettait de déterminer ou d'identifier un risque de décès chez un sujet infecté par un virus respiratoire. Ainsi, et dans un contexte où la médecine personnalisée prend de plus en plus d'importance, les sujets présentant un risque accru de mortalité pourraient bénéficier d'une prise en charge individualisée. De plus, en considérant la prévalence mondiale des virus respiratoires tels que le SARS-CoV-2 et des maladies associées, notamment leurs formes graves, il est primordial de mettre à disposition l'arsenal le plus complet possible pour déterminer de manière rapide et efficace le risque de décès que présentent les patients infectés par ces virus.

Le procédé objet de l'invention présente l'avantage de pouvoir facilement évaluer le risque accru de décès d'un sujet, par exemple un patient admis en établissement de santé tel qu'un service de réanimation ou aux urgences, en disposant d'un biomarqueur facilement mesurable et dont la mesure peut être faite directement dans l'établissement de santé l'accueillant ou dans un laboratoire de proximité. De plus, la mesure du biomarqueur ADGRE3, tout comme celle des biomarqueurs additionnels de l'invention, est tout à fait adaptée pour être réalisée par des automates d'analyse ou par des tests dits rapides.

Par « biomarqueur » ou « marqueur », on entend une caractéristique biologique mesurable objectivement et qui représente un indicateur des processus biologiques normaux ou pathologiques ou de réponse pharmacologique à une intervention thérapeutique. Au sens de la présente description, les biomarqueurs sont des gènes et leur niveau d'expression est détectable en particulier au niveau des transcrits, notamment des transcrits ARNm.

Le gène ADGRE3 (également connu sous le nom EMR3) est localisé sur le chromosome 19 de la position 14,619,117 à la position 14,674,844 (GRCh38/hg38), soit 55728 pb. Ce gène code pour un membre de la famille des récepteurs transmembranaires à sept domaines (TM7) de classe B qui sont exprimés principalement par les cellules du système immunitaire. Les membres de cette famille sont caractérisés par une région extracellulaire étendue avec un nombre variable de domaine N-terminaux de type facteur de croissance épidermique (EGF) couplés à un domaine TM7 via un domaine espaceur de type mucine. Ce gène est étroitement lié au gène codant le récepteur 2 des hormones de type mucine contenant une molécule de type EGF sur le chromosome 19. L'épissage alternatif de ADGRE3 donne lieu à des multiples variantes de transcription codant pour différentes isoformes.

La séquence nucléotidique du gène ADGRE3 est connue de l'homme du métier et est accessible auprès du NCBI sous la référence NC_000019.10 (assemblage GRCh38.p14).

L'expression « risque de décès chez un sujet » fait référence au risque que le sujet a de succomber dans les jours suivants l'infection par un virus respiratoire ou dans les jours suivants l'admission en établissement de santé consécutive à l'infection. On parle notamment de risque de décès accru lorsque le sujet présente un risque statistiquement significatif de décès dans les jours qui suivent l'infection, généralement par rapport à des sujets infectés dont on sait qu'ils ont survécus ou à des sujets non infectés.

De manière avantageuse, le procédé selon l'invention permet de déterminer le risque de décès accru chez un sujet dans les 37 jours suivants le jour où l'infection est avérée, également appelés jours post-infection, par exemple via un test de détection d'un virus respiratoire.

Ainsi, le risque de décès correspond au risque de décès du sujet dans les 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36 ou 37 jours suivants l'infection, également appelés jours post infection. De préférence, le risque de décès correspond au risque de décès du sujet dans les 16 jours, 23 jours, 30 jours, 31 jours, 32 jours, 33 jours, 34 jours, 35 jours, 36 jours, ou les 37 jours post infection du sujet. De préférence encore, le risque de décès correspond au risque de décès dans les 37 jours post-infection.

Selon un mode de réalisation particulier, le risque de décès peut également correspondre au nombre de jours suivants l'admission en établissement de santé, également appelés jours post-admission, étant entendu qu'au moment de l'admission les sujets sont déjà infectés par un virus respiratoire depuis généralement plusieurs jours. Ainsi, selon ce mode de réalisation, le risque de décès correspond au risque de décès du sujet dans les 2, 3, 4, 5, 6, 7, 8, 9, 10, 11, 12, 13, 15, 15, 16, 17, 18, 19, 20, 21, 22, 23, 24, 25, 26, 27 ou 28 jours post admission en établissement de santé du sujet. De préférence, le risque de décès correspond au risque de décès du sujet dans les 7 jours, 14 jours, 21 jours, 22 jours, 23 jours, 24 jours, 25 jours, 26 jours, 27 jours, ou les 28 jours post admission du sujet. De préférence encore, le risque de décès correspond au risque de décès dans les 28 jours post admission.

Au sens de la présente description, l'établissement de santé désigne un hôpital ou une clinique, de préférence un service des urgences, un service de réanimation, une unité de soins intensifs (USI) ou une unité de soins continus, ou encore une structure médicalisée pour personnes âgées, par exemple un EHPAD.

Le terme « sujet » désigne un être humain et de préférence, le sujet est un patient. Le patient est une personne entrée en contact avec un professionnel de santé, notamment un médecin, une structure médicale ou un établissement de santé.

Selon un mode de réalisation particulier, le sujet est un patient au sein d'un établissement de santé, de préférence au sein d'un hôpital, de préférence encore au sein du service des urgences, du service de réanimation, en unité de soins intensifs (USI) ou en unité de soins continus, et tout particulièrement un patient en USI.

Par « virus respiratoire », on entend un virus qui infecte les voies respiratoires et/ou les poumons. De tels virus se retrouvent classiquement dans les échantillons prélevés dans le nez, la gorge et/ou la bouche d'un sujet, notamment dans les échantillons nasaux ou naso-pharyngés (qui nécessitent un prélèvement plus profond dans le nez), les échantillons oro-pharyngés (qui nécessitent un prélèvement au fond de la gorge) ou la salive.

Par l'expression « sujet infecté par un virus respiratoire », on entend un sujet dont le test de détection de la présence d'un virus respiratoire donne un résultat positif. Ces sujets, notamment ceux développant les formes les plus graves de la maladies, sont des sujets pour lesquels il est d'autant plus pertinent de mettre en œuvre le procédé selon l'invention mesurant le niveau d'expression de ADGRE3.

A l'inverse, un sujet non-infecté par un virus respiratoire est un sujet dont le test de détection de la présence d'un virus respiratoire est négatif.

A titre d'exemple de virus respiratoire, on peut citer les coronavirus saisonniers, le virus SARS-CoV-2 (« Severe Acute Respiratory Syndrome Coronavirus-2 »), quels que soient ses variants, le virus de la grippe, le virus respiratoire syncytial (VRS), les rhinovirus, les métapneumovirus, les virus parainfluenza et les adénovirus. A ce jour, les variants connus du virus SARS-CoV-2, sont, notamment ses variants dits anglais, brésilien, sud-africain, américain, indien ou Omicron. La connaissance de ces variants et leurs dénomination évoluent et l'invention est applicable à chacun d'entre eux (https://www.who.int/en/activities/tracking-SARS-CoV-2-variants/).

La découverte du « variant anglais » de SARS-CoV-2 remonte au 20 septembre 2020, dans le Kent (Sud-Est de l'Angleterre). Le 14 décembre, le Royaume-Uni a signalé à l'Organisation mondiale de la santé (OMS) la circulation de ce variant. Baptisé dans un premier temps VUI 202012/01 (pour Variant Under Investigation, année 2020, mois 12, variant 01), il est rapidement renommé, le 18 décembre 2020, VOC 202012/01 (pour Variant Of Concern, littéralement « variant préoccupant»), puis variant Alpha. Il fait partie du lignage B.1.1.7 dans l'arbre phylogénétique et comporte la délétion 69-70, encore désignée ΔH69/Λ70. Le variant brésilien P.1 (variant Gamma) est un descendant du lignage B.1.1.28. Ce variant brésilien P.1 renferme de nombreuses mutations, en particulier les mutations E484K, K417T et N501Y. Le variant japonais qui dérive d'un lignage présent au Brésil (B.1.1.28) renferme un nombre très élevé de changements génétiques. Il comporte douze mutations d'acides aminés dans la protéine spike, notamment les mutations N501Y, E484K, K417T. Le variant sud-africain (variant Beta) baptisé 501Y.V2 et appartenant au lignage B. 1.351 renferme lui aussi différentes mutations dont trois, K417N, E484K et N501Y, localisées dans le domaine RBD de la protéine spike, le domaine de fixation au récepteur. Le variant Indien, baptisé variant Delta qui appartient au lignage B.1.617.2 et renferme les mutations S417N et S484K. Enfin, il y a également le variant Omicron identifié en novembre 2021 et qui appartient au lignage B.1.1.529.

Selon un mode de réalisation particulier, le virus respiratoire est un coronavirus, en particulier le SARS-CoV-2 ou un de ses variants tels que par exemple les variants Alpha, Beta, Gamma, Delta ou Omicron.

Les expressions « test de détection d'un virus respiratoire », « test de diagnostic d'un virus respiratoire » ou « test de détection de la présence d'une infection par un virus respiratoire », sont synonymes et font référence à tout test connu de l'homme du métier permettant d'apporter une telle conclusion, notamment les tests de détection de l'ADN ou de l'ARN dudit virus respiratoire, tels que les tests PCR, les tests antigéniques ou encore les auto-tests.

Par « échantillon biologique », on se réfère ici à tout échantillon provenant d'un sujet, et pouvant être de différentes natures, comme le sang ou ses dérivés, les expectorations, l'urine, les selles, la peau, le liquide céphalo-rachidien, le liquide de lavage broncho-alvéolaire, le liquide de ponction de la cavité abdominale, la salive, les sécrétions gastriques, le sperme, le liquide séminal, les larmes, la moelle épinière, ganglion du nerf trijumeau, tissu adipeux, tissu lymphoïde, tissu placentaire, tissu du tractus gastro-intestinal, tissu du tractus génital, ou le tissu du système nerveux central.

En particulier, l'échantillon biologique peut être un fluide biologique, comme un échantillon de sang ou un échantillon dérivé du sang, qui peut notamment être choisi parmi le sang total (tel que collecté par voie veineuse, c'est-à-dire contenant les cellules blanches et rouges, les plaquettes et le plasma), le plasma, le sérum, ainsi que tous les types de cellules extraites à partir du sang, comme par exemple les cellules mononuclées sanguines périphériques (ou PBMC, contenant les lymphocytes B, les lymphocytes T, les cellules NK, les cellules dendritiques et les monocytes), des sous-populations de cellules B, des monocytes purifiés, ou encore des neutrophiles.

Selon un mode de réalisation préféré, l'échantillon biologique mis en œuvre dans le procédé selon l'invention est un échantillon de sang, de préférence un échantillon de sang total.

Au sens de la présente description, l'échantillon biologique d'un sujet, à savoir celui d'un sujet infecté dont on souhaite déterminer le risque de décès, correspond à l'échantillon biologique à tester, ou échantillon test, par opposition à l'échantillon de référence utilisé comme comparatif.

Au sens de la présente description, l'expression « valeur de référence » ou « valeur de référence prédéterminée », est synonyme des expressions « valeur contrôle » ou « valeur seuil », et sert de point de comparaison pour déterminer si le niveau d'expression d'un gène cible est diminué ou augmenté.

Selon le procédé objet de l'invention, le risque de décès chez un sujet infecté par un virus respiratoire est notamment déterminé au travers de la mise en œuvre d'une étape de mesure du niveau d'expression du gène ADGRE3.

La mesure du niveau d'expression d'un gène est bien connue de l'homme du métier et consiste notamment à quantifier au moins un produit d'expression du gène. Le produit d'expression d'un gène, au sens de la présente invention, peut être toute molécule biologique issue de l'expression dudit gène. Plus particulièrement, le produit d'expression du gène peut être un transcrit.

Par « transcrit », on entend les ARN, et en particulier les ARN messagers (ARNm), issus de la transcription du gène. Plus précisément, les transcrits sont les ARN produits par la transcription d'un gène suivi des modifications post-transcriptionnelles des formes pré-ARN.

Selon un mode de réalisation préféré, la mesure du niveau d'expression de ADGRE3, et éventuellement celle d'un ou plusieurs gènes additionnels tels que listés ci-après, est réalisée au niveau ARN, en particulier au niveau messager (ARNm), dans un échantillon biologique d'un sujet infecté par un virus respiratoire. Selon ce mode de réalisation, la mesure du niveau d'expression du gène ADGRE3 concerne donc la détermination du niveau d'ARNm dudit gène.

Les transcrits du gène ADGRE3 sont connus de l'homme du métier. On cite à titre d'exemple les transcrits ayant les références suivantes dans la base de donnée NCBI: XM_011528374.3 (2298nt), NM_001289159.2 (1971nt), NM_001289158.2 (2193 nt), XM_047439546.1 (2151nt) et NM_032571.5 (2349nt).

Comme mentionné précédemment, la mesure du niveau d'expression d'un gène est bien connue de l'homme du métier. Dans le cas d'un transcrit tel que par exemple l'ARNm, la mesure peut être réalisée par une méthode directe, selon tout procédé connu de l'homme du métier permettant de déterminer la présence dudit transcrit dans l'échantillon biologique, ou par détection indirecte du transcrit après transformation de ce dernier en ADN, ou après amplification dudit transcrit ou après amplification de l'ADN obtenu après transformation dudit transcrit en ADN. De nombreuses méthodes existent pour la détection des acides nucléiques et sont bien connues de l'homme du métier (voir par exemple Kricka et al., Clinical Chemistry, 1999, n° 45(4), p.453-458 ; Relier G. H. et al., DNA Probes, 2nd Ed., Stockton Press, 1993, sections 5 et 6, p. 173-249).

A titre d'exemple, l'expression du gène peut être déterminée de la manière suivante :
(1) extraction des ARN totaux d'un échantillon sanguin ou des PBMCs et réalisation d'une étape de transcription inverse afin d'obtenir les différents ADN complémentaires des différents ARN messagers initialement présents dans l'échantillon ou les PBMCs (ou ADNc),
(2) amplification spécifique des ADNc. Dans ce cas, le réactif spécifique utilisé comprend au moins une amorce d'amplification spécifique du gène. Cette étape peut être réalisée par une réaction d'amplification de type PCR ou par tout autre technique d'amplification appropriée,
(3) détermination de l'expression du gène en quantifiant les ADNc.

L'expression des gènes peut notamment être mesurée par Reverse Transcription-Polymerase Chain Reaction ou RT-PCR, de préférence par RT-PCR quantitative ou RT-qPCR (par exemple en utilisant la technologie FilmArray^{®} ou la plateforme Biomark^{™} de Fluidigm), par séquençage (de préférence par séquençage haut débit) ou par des techniques d'hybridation (par exemple avec des micropuces d'hybridation ou par des techniques du type NanoString^{®} nCounter^{®}). Toutes ces méthodes sont également bien connues de l'homme du métier et il n'est pas nécessaire de les détailler ici.

Selon un mode de réalisation particulier, la mesure de l'expression de ADGRE3 est réalisée par une méthode de détection moléculaire, notamment par RT-PCR, par séquençage ou par hybridation. De préférence, la mesure de l'expression est réalisée par RT-PCR, et notamment par RT-qPCR.

Selon un mode de réalisation particulier, le niveau d'expression du gène ADGRE3 est mesuré par détection quantitative RT-qPCR des transcrits ARNm dudit gène. L'homme du métier est tout en fait en mesure de déterminer les amorces nécessaire à l'amplification d'au moins un transcrit du gène ADGRE3, et éventuellement des gènes additionnels, pour déterminer le(s) niveau(x) d'expression.

La mesure du niveau d'expression permet de déterminer la quantité d'un ou plusieurs transcrits de ADGRE3 dans l'échantillon biologique ou également d'en donner une valeur dérivée.

Ainsi, selon un mode de réalisation particulier, le niveau d'expression du gène ADGRE3 est une valeur dérivée de la quantité de ses transcrits, notamment ARNm. A titre d'exemple, une valeur dérivée de la quantité des transcrits peut par exemple être la concentration absolue, calculée grâce à une courbe de calibration obtenue à partir de dilutions successives d'une solution d'amplicons de concentration connue. La valeur dérivée peut également correspondre à la valeur de la quantité normalisée et calibrée, comme le CNRQ (Calibrated Normalized Relative Quantity, (Hellemans et al (2007), Genome biology 8(2): R19), qui intègre les valeurs d'un échantillon de référence (ou d'un calibrateur) et d'un ou plusieurs gènes de ménage (appelés également gènes de référence). A titre d'exemples de gènes de ménage, on peut citer les gènes DECR1, HPRT1, PPIB, RPLPO, PPIA, GLYR1, RANBP3, 18S, B2M, TBP, GAPDH et ACTB.

Selon un mode de réalisation particulier, l'expression de ADGRE3 est normalisée par rapport à l'expression d'un ou plusieurs gènes de ménage (ou gènes de référence) selon les méthodes connues de l'homme du métier. Ainsi, l'expression est normalisée en utilisant un ou plusieurs des gènes de ménage suivants : DECR1 (localisation chromosomique: chr8, 90001352-90053633), HPRT1 (localisation chromosomique: chrX, 134452842-134520513) et PPIB (localisation chromosomique: chr15:64155812-64163205), RPLPO (localisation chromosomique: chr12, 120196699-120201111), PPIA (localisation chromosomique: chr7 , 44795960-44803117), GLYR1 (localisation chromosomique: chr16, 4803203-4847288), RANBP3 (localisation chromosomique: chr19, 5916139-5978140), B2M (localisation chromosomique: chr15, 44711492-44718145), TBP (localisation chromosomique : chr6, 170554369-170572859), GAPDH (localisation chromosomique: chr12, 6534517-6538371) et ACTB (localisation chromosomique : chr14 , 5527148-5530601). Les localisations chromosomiques sont données selon le GRCh38/hg38. De préférence, l'expression est normalisée en utilisant un ou plusieurs des gènes de ménage choisis parmi : DECR1, HPRT1, PPIB, GAPDH, ACTB et leurs combinaisons, de préférence encore, choisis parmi DECR1, HPRT1, PPIB et leurs combinaisons.

Dans un tel cas, le niveau de référence utilisé est également normalisé au préalable, de la même manière. La normalisation, que ce soit pour le niveau de référence ou pour le niveau de transcrits de l'échantillon biologique à tester, est réalisée avant la comparaison, notamment avant le calcul d'un rapport entre le niveau de transcrits dudit échantillon à tester et le niveau de référence. Lorsqu'une valeur seuil différente d'un niveau de référence est utilisée pour émettre une conclusion, il pourra être tenu compte de cette normalisation, pour le choix de la valeur seuil.

Dans le cas où le niveau de transcrits de ADGRE3 est normalisé par rapport au niveau de transcrits d'un ou plusieurs gènes de ménage, bien entendu, cela implique que le procédé selon l'invention inclut la détermination du niveau de transcrits du ou des gènes de ménage utilisé(s) pour la normalisation.

D'une manière générale, dans le procédé selon l'invention, quels que soient ses modes de réalisation, le niveau d'expression de ADGRE3, de préférence l'expression normalisée, dans l'échantillon biologique du sujet est comparé à une valeur de référence ou à l'expression du même gène, de préférence l'expression normalisée, obtenue dans un échantillon biologique de référence.

Selon un mode de réalisation particulier, le niveau d'expression du gène ADGRE3 est déterminé à partir d'un échantillon biologique d'un sujet et il peut être conclu à un risque de décès accru chez ledit sujet lorsque la comparaison du niveau d'expression dudit gène ADGRE3 avec une valeur de référence prédéterminée, montre qu'il y a une différence significative avec ladite valeur de référence correspondant audit gène. Plus précisément, ladite différence correspond à un niveau de transcrits, notamment ARNm, pour l'échantillon biologique à tester qui est inférieur à celui correspondant à la valeur de référence. En d'autres termes, il pourra être conclu à un risque accru de décès chez ledit sujet lorsqu'une sous-expression du gène ADGRE3 est mise en évidence.

Par « sous-expression », on entend une diminution significative du niveau d'expression par rapport à une valeur de référence. L'homme du métier est en mesure de déterminer le test statistique à utiliser pour déterminer cette valeur de référence avec laquelle le niveau d'expression de ADGRE3 doit être comparé. Les exemples de réalisation présentent une des méthodes possibles.

De manière avantageuse, selon une première variante, ladite valeur de référence correspond à un niveau d'expression de référence du gène ADGRE3 qui est le niveau de transcrits dudit gène obtenu à partir d'un échantillon biologique d'un sujet ne présentant aucune infection par un virus respiratoire. Selon cette variante, la valeur de référence peut également correspondre à la moyenne du niveau d'expression de transcrits de ADGRE3 obtenue à partir d'échantillons biologiques issus d'une population de sujets ne présentant aucune infection par un virus respiratoire.

Selon une deuxième variante, la valeur de référence correspond à un niveau d'expression de référence du gène ADGRE3 qui est le niveau de transcrits dudit gène obtenu à partir d'un échantillon biologique d'un sujet infecté par un virus respiratoire mais dont on sait qu'il a survécu après l'infection, notamment dans les 37 jours suivants l'infection. Selon cette variante, la valeur de référence peut également correspondre à la moyenne du niveau d'expression de transcrits de ADGRE3 obtenue à partir d'échantillons biologiques issus d'une population de sujets infectés par un virus respiratoire mais dont on sait qu'ils ont survécus après l'infection, notamment dans les 37 jours suivants l'infection.

De manière préférée, la différence entre le niveau de transcrits du gène ADGRE3 déterminé dans l'échantillon biologique test et le niveau de référence dudit gène (valeur de référence), correspond au fait que le niveau de transcrits dudit gène déterminé dans l'échantillon test est diminué de manière significative, en particulier d'au moins 10%, d'au moins 20%, d'au moins 30 %, d'au moins 40%, d'au moins 50%, d'au moins 60%, d'au moins 70%, d'au moins 80% ou d'au moins 90% par rapport au niveau d'expression de référence, ou valeur de référence, dudit gène ADGRE3.

La diminution considérée comme pertinente pour apporter une conclusion sera fonction de la valeur de référence considérée, et notamment du niveau de référence utilisé comme valeur de référence, et peut être adaptée par l'homme du métier. En particulier, si la valeur de référence correspond au niveau de référence du gène ADGRE3 qui est le niveau de transcrits dudit gène chez un sujet ne présentant aucune infection par un virus respiratoire, chez une population de sujets ne présentant aucune infection par un respiratoire, ou chez un sujet ou une population de sujets présentant une infection par virus respiratoire mais ayant survécus dans les 37 jours suivants l'infection, il pourra suffire que le niveau de transcrits ADGRE3 déterminé dans l'échantillon biologique test soit simplement inférieur à ladite valeur seuil de référence.

Selon l'invention, le procédé permet de conclure à un risque accru de décès du sujet lorsqu'une sous-expression de ADGRE3 est mise en évidence dans l'échantillon biologique à tester.

Le procédé selon l'invention peut ainsi comprendre les étapes suivantes :
- déterminer le niveau d'expression du gène ADGRE3 par mesure de la quantité d'au moins un transcrit de ADGRE3 dans un échantillon biologique du sujet,
- comparer la quantité dudit transcrit déterminée pour ledit échantillon biologique ou une valeur dérivée de cette quantité, à une valeur de référence prédéterminée à partir d'un échantillon de référence, et
- établir une conclusion sur la présence d'un risque accru de décès lorsque le résultat de la comparaison montre une diminution de l'expression du gène ADGRE3.

Selon ce mode de réalisation, l'échantillon de référence peut être par exemple un échantillon provenant d'un sujet ou un mélange d'échantillons de plusieurs sujets, lesdits sujets étant non-infectés par un virus respiratoire. La valeur de référence peut notamment correspondre à une valeur moyenne du niveau d'expression de ADGRE3 mesurée à partir de plusieurs d'échantillons issus chacun de différents sujets non-infectés par un virus respiratoire ou infectés par un virus respiratoire mais dont on sait qu'ils ont survécu, notamment dans les 28 jours suivants l'admission en établissement de santé ou dans les 37 jours post infection.

Selon une variante préférée de ce mode de réalisation, l'échantillon de référence est de même nature que l'échantillon biologique à tester ou tout du moins d'une nature compatible pour constituer une référence quant à la détermination du niveau d'expression de ADGRE3.

Une « comparaison » ou une vérification d'une « différence » entre deux valeurs, ou niveaux de valeurs, peut être effectuée par toute technique connue. La comparaison ou la vérification d'une « différence » peut impliquer le calcul d'un rapport ou d'une différence.

Dans le cadre de l'invention, l'émission d'une conclusion quant au risque de décès chez le sujet dont est issu l'échantillon biologique test, peut également être effectuée par toute technique automatisée, réalisée par un ordinateur ou assistée par ordinateur.

Le procédé tel que décrit précédemment, dans tous ses modes de réalisations, peut également comprendre, outre l'étape de mesure de l'expression du gène ADGRE3, une étape de mesure de l'expression d'un ou plusieurs gènes additionnels.

Ainsi, selon ce mode de réalisation particulier, le ou les gènes additionnels sont choisis parmi C3AR1, CD177, OAS2, CIITA, IL-10, IL1R2, CD74, TDRD9 et leurs combinaisons. Selon ce mode de réalisation, le procédé comprend également une étape de comparaison du niveau d'expression du ou des gènes additionnels avec une valeur de référence du niveau d'expression respectif desdits gènes additionnels. Les valeurs de référence des gènes additionnels sont telles que définies précédemment avec ADGRE3.

Les localisations chromosomiques des gènes additionnels sont données dans le tableau 1 ci-dessous :

**[Tableau 1]**

| Gène (Nom) | Localisation chromosomique (GRCh38/hg38) | Numéro d'accession Ensembl & NCBI |
|---|---|---|
| C3AR1 (Complément C3a du récepteur 1) | Chromosome 12: 8,056,844-8,066,359 | ENSG00000171860 NC_000012.12 |
| CD177 (molécule CD177) | Chromosome 19: 43,353,686-43,363,172 | ENSG00000204936 NC_000019.10 |
| OAS2 (2'5'-oligoadénylate synthétase 2) | Chromosome 12: 112,978,395-113,011,723 | ENSG00000111335 NC_000012.12 |
| CIITA (transactivateur de classe II du Complexe Majeur d'Histocompatibilité) | Chromosome 16: 10,866,222-10,943,021 | ENSG00000179583 NC_000016.10 |
| IL-10 (Interleukine 10) | Chromosome 1: 206,767,602-206,774,541 | ENSG00000136634 NC_000001.11 |
| IL1R2 (Récepteur 2 de l'interleukine 1) | Chromosome 2: 101,991,960-102,028,544 | ENSG00000115590 NC_000002.12 |
| CD74 (molecule CD74) | Chromosome 5: 150,401,639-150,412,910 | ENSG00000019582 NC_000005.10 |
| TDRD9 (tudor domain containing 9) | Chromosome 14: 103,928,456-104,052,667 | ENSG00000156414 NC_000014.9 |

Ainsi, il pourra être conclu à un risque accru de décès chez ledit patient lorsque la comparaison du niveau d'expression du gène ADGRE3, notamment des transcrits ARNm dudit gène, avec une valeur de référence prédéterminée montre qu'il y a une diminution du niveau d'expression ou une sous expression, et lorsque la comparaison du niveau d'expression des gènes additionnels ci-dessous, notamment des transcrits ARNm desdits gènes, avec une valeur de référence de leur niveau d'expression respectif, montre qu'il y a :
- une augmentation du niveau d'expression ou une surexpression de C3AR1, et/ou
- une augmentation du niveau d'expression ou une surexpression de CD177, et/ou
- une augmentation du niveau d'expression ou une surexpression de OAS2, et/ou
- une diminution du niveau d'expression ou une sous expression de CIITA, et/ou
- une augmentation du niveau d'expression ou une surexpression de IL-10, et/ou
- une augmentation du niveau d'expression ou une surexpression de IL1R2, et/ou
- une diminution du niveau d'expression ou une sous expression de CD74, et/ou
- une augmentation du niveau d'expression ou une surexpression de TDRD9.

Selon une variante de ce mode de réalisation, le procédé comprend concernant la mesure de l'expression génique, outre l'étape de mesure de l'expression du gène ADGRE3, uniquement une étape de mesure de l'expression d'un ou plusieurs gènes additionnels choisis parmi C3AR1, CD177, OAS2, CIITA, IL-10, IL1R2, et leurs combinaisons, de préférence parmi C3AR1, CD177, CIITA, IL-10, IL1R2 et leurs combinaisons.

Selon une autre variante de ce mode de réalisation particulier, le procédé comprend concernant la mesure de l'expression génique, outre l'étape de mesure de l'expression du gène ADGRE3, uniquement une étape de mesure de l'expression du gène additionnel IL-10.

Selon une autre variante de ce mode de réalisation, le procédé comprend concernant la mesure de l'expression génique, outre l'étape de mesure de l'expression du gène ADGRE3, uniquement une étape de mesure de l'expression du gène additionnel IL-10 et une étape de mesure de l'expression d'au moins un autre gène additionnel choisi parmi C3AR1, CD177, OAS2, CIITA, IL1R2, CD74, TDRD9 et leurs combinaisons, de préférence, choisi parmi C3AR1, CD177, OAS2, CIITA et leurs combinaisons, et de préférence encore, choisi parmi C3AR1, CIITA, et leurs combinaisons.

Selon une autre variante de ce mode de réalisation particulier, le procédé comprend concernant la mesure de l'expression génique, outre l'étape de mesure de l'expression du gène ADGRE3, uniquement une étape de mesure de l'expression du gène additionnel CIITA.

Selon une autre variante de ce mode de réalisation particulier, le procédé comprend concernant la mesure de l'expression génique, outre l'étape de mesure de l'expression du gène ADGRE3, uniquement une étape de mesure de l'expression du gène additionnel CIITA et une étape de mesure de l'expression d'au moins un autre gène additionnel choisi parmi C3AR1, CD177, OAS2, IL-10, IL1R2, CD74, TDRD9 et leurs combinaisons, de préférence choisi parmi C3AR1, CD177, OAS2, IL-10 et leurs combinaisons, et de préférence encore, choisi parmi C3AR1, IL-10 et leurs combinaisons.

Selon une autre variante de ce mode de réalisation particulier, le procédé comprend concernant la mesure de l'expression génique, outre l'étape de mesure de l'expression du gène ADGRE3, uniquement une étape de mesure de l'expression du gène additionnel CD74.

Selon une autre variante de ce mode de réalisation particulier, le procédé comprend concernant la mesure de l'expression génique, outre l'étape de mesure de l'expression du gène ADGRE3, uniquement une étape de mesure de l'expression du gène additionnel CD74 et une étape de mesure de l'expression d'au moins un autre gène additionnel choisi parmi C3AR1, CD177, OAS2, IL-10, IL1R2, CIITA, TDRD9 et leurs combinaisons, de préférence parmi C3AR1, CD177, OAS2, IL-10, IL1R2, CIITA et leurs combinaisons, et de préférence encore, parmi C3AR1, IL-10, CIITA et leurs combinaisons.

Selon une autre variante de ce mode de réalisation, le procédé comprend concernant la mesure de l'expression génique, outre l'étape de mesure de l'expression du gène ADGRE3, uniquement une étape de mesure de l'expression du gène additionnel OAS2.

Selon une autre variante de ce mode de réalisation particulier, le procédé comprend concernant la mesure de l'expression génique, outre l'étape de mesure de l'expression du gène ADGRE3, uniquement une étape de mesure de l'expression du gène additionnel OAS2 et une étape de mesure de l'expression d'au moins un autre gène additionnel choisi parmi C3AR1, CD177, CD74, IL-10, IL1R2, CIITA, TDRD9 et leurs combinaisons, de préférence choisi parmi C3AR1, CD177, IL-10, IL1R2, CIITA et leurs combinaisons, et de préférence encore choisi parmi C3AR1, IL-10, CIITA et leurs combinaisons.

Selon une autre variante de ce mode de réalisation, le procédé comprend concernant la mesure de l'expression génique, outre l'étape de mesure de l'expression du gène ADGRE3, uniquement une étape de mesure de l'expression du gène additionnel C3AR1.

Selon une autre variante de ce mode de réalisation particulier, le procédé comprend concernant la mesure de l'expression génique, outre l'étape de mesure de l'expression du gène ADGRE3, uniquement une étape de mesure de l'expression du gène additionnel C3AR1 et une étape de mesure de l'expression d'au moins un autre gène additionnel choisi parmi OAS2, CD177, CD74, IL-10, IL1R2, CIITA, TDRD9 et leurs combinaisons, et de préférence choisi parmi OAS2, CD177, IL-10, IL1R2, CIITA et leurs combinaisons, et de préférence encore choisi parmi IL-10, CIITA et leurs combinaisons.

Selon une autre variante de ce mode de réalisation, le procédé comprend concernant la mesure de l'expression génique, outre l'étape de mesure de l'expression du gène ADGRE3, uniquement une étape de mesure de l'expression du gène additionnel IL1R2.

Selon une autre variante de ce mode de réalisation particulier, le procédé comprend concernant la mesure de l'expression génique, outre l'étape de mesure de l'expression du gène ADGRE3, uniquement une étape de mesure de l'expression du gène additionnel IL1R2 et une étape de mesure de l'expression d'au moins un autre gène additionnel choisi parmi C3AR1, CD177, CD74, IL-10, OAS2, CIITA, TDRD9 et leurs combinaisons, de préférence choisi parmi C3AR1, CD177, IL-10, OAS2, CIITA et leurs combinaisons, et de préférence encore choisi parmi C3AR1, IL-10, CIITA et leurs combinaisons.

Selon une autre variante de ce mode de réalisation, le procédé comprend concernant la mesure de l'expression génique, outre l'étape de mesure de l'expression du gène ADGRE3, uniquement une étape de mesure de l'expression du gène additionnel TDRD9.

Selon une autre variante de ce mode de réalisation, le procédé comprend concernant la mesure de l'expression génique, outre l'étape de mesure de l'expression du gène ADGRE3, uniquement une étape de mesure de l'expression du gène additionnel TDRD9 et une étape de mesure de l'expression d'au moins un autre gène additionnel choisi parmi C3AR1, CD177, OAS2, IL-10, IL1R2, CIITA et leurs combinaisons, de préférence parmi C3AR1, CD177, OAS2, IL-10, IL1R2, CIITA et leurs combinaisons, et de préférence encore, parmi C3AR1, OAS2, IL-10, CIITA et leurs combinaisons.

Selon une autre variante de ce mode de réalisation, le procédé comprend, outre l'étape de mesure de l'expression du gène ADGRE3, une étape de mesure de l'expression des 8 gènes additionnels suivants : C3AR1, CD177, OAS2, CIITA, IL-10, IL1R2, CD74 et TDRD9.

Selon une variante de réalisation particulièrement préférée, le procédé selon l'invention, comprend conjointement à la mesure de l'expression du gène ADGRE3, la mesure de l'expression des gènes additionnels présentés dans le tableau 2 :

**[Tableau 2]**

| | |
|---|---|
| | C3AR1, CIITA C3AR1, IL-10 |
| Combinaisons de 2 gènes additionnels | C3AR1, OAS2 CIITA, IL-10 CIITA, OAS2 IL-10, OAS2 |
| Combinaisons de 3 gènes additionnels | C3AR1, CIITA, IL-10 C3AR1, CIITA, OAS2 C3AR1, IL-10, OAS2 CIITA, IL-10, OAS2 |
| Combinaison de 4 gènes additionnels | C3AR1, CIITA, IL-10, OAS2 C3AR1, CD74, IL-10, TDRD9 |

Pour toutes les variantes de réalisation décrites précédemment, le procédé comprend une étape de conclusion quant au risque accru de décès sur la base de la mesure des niveaux d'expression desdits gènes, et de la comparaison des niveaux ainsi mesurés (augmentation ou diminution) avec des valeurs de référence déterminées comme décrit précédemment.

Un autre objet de l'invention concerne un kit pour la mesure in vitro ou ex vivo du niveau d'expression du gène ADGRE3 dans un échantillon biologique d'un sujet, ledit kit comprenant au moins un moyen de détermination du niveau d'expression de ADGRE3 dans ledit échantillon biologique.

Les moyens de détermination du niveau d'expression du gène ADGRE3 sont connus de l'homme du métier et peuvent être des outils ou réactifs spécifiques permettant de mesurer ladite expression dans un échantillon biologique. Il peut par exemple s'agir d'amorces ou des sondes.

Les modes de réalisation spécifiques ou préférés décrits en lien avec les procédés selon l'invention s'appliquent, bien entendu, aux kits et utilisations faisant l'objet de l'invention.

On entend par « amorce » ou « amorce d'amplification », un fragment nucléotidique pouvant être constitué de 5 à 100 nucléotides, de préférence de 15 à 30 nucléotides, et possédant une spécificité d'hybridation avec une séquence nucléotidique cible, dans des conditions déterminées pour l'initiation d'une polymérisation enzymatique, par exemple dans une réaction d'amplification enzymatique de la séquence nucléotidique cible. Généralement, on utilise des « couples d'amorces », constitués de deux amorces. Lorsque l'on souhaite réaliser l'amplification de plusieurs biomarqueurs (e.g des gènes) différents, plusieurs couples d'amorces différents sont de préférence utilisés, ayant préférentiellement chacun une capacité à s'hybrider spécifiquement avec un biomarqueur différent.

L'homme du métier est ainsi en mesure à partir de la séquence du gène et notamment des transcrits correspondants, de déterminer les amorces et les sondes nécessaires pour l'amplification, notamment pour l'amplification d'un ou plusieurs transcrits ARNm dudit gène.

On entend par « sonde » ou « sonde d'hybridation », un fragment nucléotidique constitué typiquement de 5 à 100 nucléotides, de préférence de 15 à 90 nucléotides, de manière encore plus préférée de 15 à 35 nucléotides, possédant une spécificité d'hybridation dans des conditions déterminées pour former un complexe d'hybridation avec une séquence nucléotidique cible. La sonde comporte également un rapporteur (tel qu'un fluorophore, une enzyme ou tout autre système de détection), qui va permettre la détection de la séquence nucléotidique cible. Dans la présente invention, la séquence nucléotidique cible peut être une séquence nucléotidique comprise dans un ARN messager (ARNm) ou une séquence nucléotidique comprise dans un ADN complémentaire (ADNc) obtenu par transcription inverse dudit ARNm. Lorsque l'on souhaite cibler plusieurs biomarqueurs (e.g. des gènes) différents, plusieurs sondes différentes sont de préférence utilisées, ayant préférentiellement chacune une capacité à s'hybrider spécifiquement avec un biomarqueur différent.

Par « hybridation », on entend le processus au cours duquel, dans des conditions appropriées, deux fragments nucléotidiques, tels que par exemple une sonde d'hybridation et un fragment nucléotidique cible, ayant des séquences suffisamment complémentaires, sont susceptibles de former un double brin avec des liaisons hydrogènes stables et spécifiques. Un fragment nucléotidique "capable de s'hybrider" avec un polynucléotide est un fragment pouvant s'hybrider avec ledit polynucléotide dans des conditions d'hybridation, qui peuvent être déterminées dans chaque cas de façon connue. Les conditions d'hybridation sont déterminées par la stringence, c'est-à-dire la rigueur des conditions opératoires. L'hybridation est d'autant plus spécifique qu'elle est effectuée à plus forte stringence. La stringence est définie notamment en fonction de la composition en bases d'un duplex sonde/cible, ainsi que par le degré de mésappariement entre deux acides nucléiques. La stringence peut également être fonction des paramètres de la réaction, tels que la concentration et le type d'espèces ioniques présentes dans la solution d'hybridation, la nature et la concentration d'agents dénaturants et/ou la température d'hybridation. La stringence des conditions dans lesquelles une réaction d'hybridation doit être réalisée dépendra principalement des sondes d'hybridation utilisées. Toutes ces données sont bien connues et les conditions appropriées peuvent être déterminées par l'homme du métier.

En général, selon la longueur des sondes d'hybridation utilisées, la température pour la réaction d'hybridation est comprise entre environ 20 et 70°C, en particulier entre 35 et 65°C dans une solution saline à une concentration d'environ 0,5 à 1 M. On réalise ensuite une étape de détection de la réaction d' hybridation.

Selon un mode de réalisation particulier, au moins une valeur de référence est stockée sur un support lisible par un ordinateur, par exemple un code barre, et/ou peut être utilisée sous la forme d'un code exécutable par un ordinateur configuré pour comparer le niveau de transcrits de ADGRE3 déterminé grâce au moyen de détermination, ou une donnée obtenue à partir dudit niveau de transcrits du gène ADGRE3, à ladite valeur de référence.

Selon ce mode de réalisation, la valeur de référence peut correspondre au niveau de transcrits, de préférence au niveau d'ARNm, de ADGRE3 chez un sujet ne présentant aucune infection par un virus respiratoire ou présentant une infection par un virus respiratoire mais ayant survécu dans les 37 jours suivants l'infection. La valeur de référence peut également correspondre à la moyenne du niveau de transcrits, de préférence au niveau d'ARNm, de ADGRE3 chez une population sujets ne présentant aucune infection par un virus respiratoire ou présentant une infection par un virus respiratoire mais ayant survécu dans les 37 jours suivants l'infection.

Selon un autre mode de réalisation, le kit comprend, en outre, au moins un niveau de référence pour le gène marqueur ADGRE3, stocké sur un support lisible par un ordinateur, par exemple un code barre et/ou utilisé sous la forme d'un code exécutable par un ordinateur configuré pour comparer le niveau de transcrits de ADGRE3 déterminé grâce au moyen de détermination audit niveau de référence, avec ledit niveau de référence qui est, de préférence, le niveau de transcrits de ADGRE3, chez un sujet non infecté par un virus respiratoire, ou chez une population de tels sujets.

Selon un autre mode de réalisation, le kit comprend en outre un échantillon contrôle positif qui est un échantillon calibré pour contenir la quantité de ADGRE3 qui correspond à la quantité ou concentration représentative du niveau d'expression mesuré dans un pool d'échantillons de sujets dont on sait qu'ils ne présentent pas d'infection par un virus respiratoire, et/ou un échantillon contrôle négatif qui est un échantillon calibré pour contenir la quantité de ADGRE3 qui correspond à la quantité moyenne mesurée dans un pool d'échantillons de sujets dont on sait qu'ils n'ont pas survécu, notamment dans les 37 jours, suite à une infection par un virus respiratoire.

Selon un autre mode de réalisation, le kit comprend en outre un échantillon contrôle positif qui est un échantillon calibré pour contenir la quantité de ADGRE3 qui correspond à la quantité ou concentration représentative du niveau d'expression mesuré dans un pool d'échantillons de sujets infectés par un virus respiratoire et dont on sait qu'ils ont survécu après 37 jours suivant l'infection, et/ou un échantillon contrôle négatif qui est un échantillon calibré pour contenir la quantité de ADGRE3 qui correspond à la quantité moyenne mesurée dans un pool d'échantillons de sujets dont on sait qu'ils n'ont pas survécu, notamment dans les 37 jours, suite à une infection par un virus respiratoire.

Le kit, dans tous ses modes de réalisation, peut également comprendre au moins un moyen additionnel de détermination du niveau d'expression d'un ou plusieurs gènes additionnels choisis parmi C3AR1, CD177, OAS2, CIITA, IL-10, IL1R2, CD74, TDRD9 et leurs combinaisons.

Selon ce mode de réalisation, le kit peut comprendre, de la même manière que définie précédemment, une valeur référence du niveau d'expression des gènes additionnels et/ou des échantillons contrôles (positifs ou négatifs) desdits gènes additionnels. Les moyens de détermination du niveau d'expression peuvent être des outils ou réactifs spécifiques tels que définis précédemment, par exemple des amorces ou des sondes.

Selon un mode de réalisation préféré, le kit peut également comprendre, outre un moyen de détermination du niveau d'expression de ADGRE3, au moins un autre moyen additionnel de détermination du niveau d'expression, à partir d'un échantillon biologique d'un sujet, d'un ou plusieurs gènes additionnels choisi parmi C3AR1, CD177, OAS2, CIITA, IL-10, IL1R2 et leurs combinaisons, de préférence choisi parmi C3AR1, OAS2, CIITA, IL-10 et leurs combinaisons.

Selon ce mode de réalisation, le kit peut également comprendre, une valeur référence du niveau d'expression des gènes additionnels et/ou des échantillons contrôles (positifs ou négatifs) desdits gènes additionnels.

Un autre objet concerne l'utilisation d'un kit de mesure *in vitro* ou *ex vivo* tel que défini précédemment pour déterminer le risque de décès chez un sujet infecté par un virus respiratoire.

L'invention concerne également les procédés de détermination *in vitro* ou in vivo pour déterminer le risque de décès chez un sujet infecté par un virus respiratoire tels que définis par la présente description, qui comprennent en outre une étape de traitement de l'infection dudit virus respiratoire.

En particulier, le traitement peut être initié, dès lors qu'il est conclu au risque de décès chez ledit sujet infecté par un virus respiratoire.

Le traitement peut consister à l'administration d'un médicament antiviral adapté. A titre de traitement antiviral, on peut notamment citer l'Iopinavir^{®}, le Ritonavir^{®}, les interferons recombinants, notamment l'interferon beta, alpha et lambda. De nombreux traitements thérapeutiques de virus respiratoires, notamment ceux responsables de la COVID-19, sont actuellement en cours de test (Canedo-Marroquín, G. et al., 2020).

Selon un mode de réalisation particulier, l'étape de traitement consiste en l'administration d'un ou plusieurs anticorps monoclonaux audit sujet. Des exemples de tels anticorps sont le casirivimab, l'imdevimab, le regdanvimab, le tixagevimab ou le cilgavimab. Des traitements préférés peuvent consister en la combinaison du casirivimab et du l'imdevimab (Ronapreve), ou en la combinaison du tixagevimab et du cilgavimab.

Un autre objet concerne un procédé comprenant les étapes suivantes :
- obtenir un échantillon biologique de sang, de préférence de sang total, d'un sujet infecté par un virus respiratoire qui est le SARS-CoV-2,
- mettre en contact ledit échantillon biologique avec des réactifs spécifiques des produits d'expression des gènes ADGRE3, C3AR1, CD177, OAS2, CIITA, IL-10, IL1R2, CD74, TDRD9, de préférence, ADGRE3, C3AR1, CD177, OAS2, CIITA, IL-10, IL1R2, et de préférence encore, ADGRE3, C3AR1, CIITA, et IL-10,
- mesurer l'expression desdits gènes cibles.

Les réactifs spécifiques des produits d'expression sont sélectionnés parmi des amorces d'amplification, des sondes d'hybridation ou des anticorps, et sont tels que définis précédemment.

Selon un mode de réalisation particulier, le procédé peut comprendre une étape d'administration d'un médicament antiviral adapté tel que défini précédemment, notamment lorsque l'expression des gènes cibles indique que le sujet présente un risque de décès dans les 37 jours.

Un autre objet concerne un procédé pour déterminer si un patient infecté par un virus respiratoire présente un risque de décès accru comprenant les étapes suivantes de :
- obtenir un échantillon biologique, de préférence de sang total, d'un patient infecté par un virus respiratoire,
- mesurer le niveau d'expression du gène ADGRE3 dans ledit échantillon biologique,
- comparer le niveau d'expression du gène ADGRE3 avec une valeur de référence obtenue à partir de patients infectés par un virus respiratoire et qui ont survécu, dans lequel, si le niveau d'expression du gène ADGRE3 est inférieur à la valeur de référence, il est déterminé que le patient présente un risque accru de décès.

Selon un mode de réalisation particulier, le procédé comprend également la mesure du niveau d'expression d'un ou plusieurs gènes additionnels tels que définis précédemment et la comparaison des niveaux mesurés avec des valeurs de références respectives de chaque gènes additionnels également obtenues à partir de patients infectés par un virus respiratoire et qui ont survécu. Dans ce cas, la détermination du risque de décès accru est faite

Enfin, un autre objet concerne un procédé comprenant la mesure quantitative, notamment par RT-qPCR, des ARNm du gène ADGRE3, et éventuellement d'un ou plusieurs gènes additionnels tels que définis précédemment, dans un échantillon biologique de sang d'un sujet infecté par un virus respiratoire tel que le SARS-CoV-2.

La présente invention est illustrée de manière non limitative à partir des exemples suivants.

### Exemples

### Matériel et méthode

### 1. Description des cohortes de patients

La cohorte RICO (REA-IMMUNO-COVID) est une étude clinique prospective observationnelle toujours en cours. Dans cette étude auxiliaire, 309 patients ont été recrutés entre Août 2020 et Août 2021 dans cinq unités de soins intensifs d'hôpitaux universitaires affiliés (Hospices Civils de Lyon, France).

Les patients présentaient tous une infection pulmonaire due au SARS-CoV-2. Les résultats de cette cohorte ont été publié précédemment (F. Venet *et al.,* 2021). Brièvement, les critères d'inclusion étaient les suivants : (1) homme ou femme ≥ 18 ans, (2) hospitalisation en unités de soins intensifs (USI) pour une infection respiratoire au SARS-CoV-2, (3) première hospitalisation en USI, (4) diagnostic positif d'infection à SARS-CoV-2 réalisé par PCR ou par une autre méthode approuvée dans au moins un échantillon respiratoire, (5) prélèvement d'échantillon de sang dans les premières 24h après l'admission en USI (J-0) réalisable et (6) patient ou proche parent ayant été informé des termes de l'étude et ne s'étant pas opposé à sa participation.

Les critères d'exclusion étaient la grossesse, les patients institutionnalisés et l'incapacité à obtenir le consentement éclairé.

A partir de cette cohorte, les échantillons ont été prélevés et analysés à J-0. Les patients étaient âgés en moyenne de 65 ans [InterQuartile Range (IQR), 57-72]) et présentaient une distribution disparate d'hommes et de femmes (68/32).

Cohorte de volontaires sains: des échantillons de sang de 49 volontaires sains ont été obtenus de manière indépendante auprès de l'Etablissement Français du Sang (EFS, Lyon, France). Les donneurs sains étaient âgés de 40 ans [IQR, 27-54] avec une répartition hommes/femmes hétérogène. Tous les échantillons ont été prélevés en avril 2020 et novembre 2021.

### 2. Analyse du transcriptome

Des échantillons de sang ont été collectés dans des tubes PAXgene^{®} (ref. 762165, PreAnalytiX GmbH Hombrechtikon Switzerland ) à J0 en suivant les recommandations du fabricant. En bref, les échantillons ont été laissés à température ambiante pendant 2h avec les réactifs présents dans les tubes avant d'être transférés à -20°C pendant au moins 24h, puis stockés à -80°C.

Les échantillons ont été testés en utilisant une poche FilmArray^{®} optimisée pour détecter des gènes impliqués dans la réponse de l'hôte, notamment les ARNm des gènes tels que ADGRE3, par nested PCR. Les poches sont analysées à l'aide de l'instrument FilmArray^{®}Torch (BioFire, USA) en suivants les recommandations du fabriquant Les résultats des niveaux d'expression sont obtenus de manière automatisée, en moins d'une heure, avant d'être compilés pour être analysés.

Les valeurs d'expression normalisées des marqueurs (par rapport aux gènes de références DECR1, HPRT1 et PPIB) ont été calculées et utilisées pour les analyses.

### 3. Mesures des marqueurs immunologiques

Le nombre de lymphocytes T CD3+ des lymphocytes T a été déterminé sur un cytomètre en flux volumétrique automatisé (Aquios CL, Beckman Coulter).

Les valeurs standardisés (Anticorps/Cellules ou Ac/C) de l'expression du HLA-DR par les monocytes (mHLA-DR) ont été obtenues avec un cytomètre en flux

(Navios, Beckman Coulter) avec les réactifs HLA-DR Quantribite (Becton Dickinson) tel que décrit précédemment (F. Venet *et al.,* 2021).

### 4. Analyses statistiques

La cohorte RICO a été divisée de manière aléatoire afin d'obtenir deux jeux de données équilibrés selon 3 paramètres : l'âge, le sexe et la mortalité. Ainsi, un premier jeu de données de 216 patients utilisé pour l'apprentissage automatique et un jeu de données test indépendant de 93 patients utilisé pour la validation des performances. Pour la description des jeux de données, les données qualitative ont été rapportées sous forme de nombres ou de fréquence et les données quantitatives ont été rapportées sous forme de médiane [intervalle IQR].

Les caractéristiques cliniques ont été comparées à l'aide du test non paramétrique de Mann-Whitney-Wilcoxon pour les variables continues, et du test exact de Fisher ou du test du chi carré (le cas échéant) pour les variables catégorielles. Le niveau de signifiance a été fixé à 5 % pour les tests bilatéraux. Les analyses statistiques ont été réalisées à l'aide du logiciel R, version 3.6.2. Les données ont été centrées et réduites pour réaliser une analyse en composantes principales non supervisée via le package FactoMineR (version 2.4).

Les gènes significativement associés à la mortalité à 28 jours dans un modèle univarié de régression logistique ont été utilisés pour construire les modèles multivariés de prédiction de la survie à 28 jours. Les modèles entrainés sont des modèles de régression logistique avec régularisation L1 (Lasso), L2(Ridge) et mixte (ElasticNet), l'analyse « Partial Least Squares-Discriminant » (PLS), et le Support Vector Machines avec kernels linéaires (linear SVM) utilisant package CARET (version 6.0-84).

Pour compenser la répartition déséquilibrée de la mortalité dans les ensembles de données, la technique de sur-échantillonnage de minorité synthétique (SMOTE) a été appliquée pour le réglage des hyper paramètres (N.V Chawla *et al.,* 2002).

Les hyper paramètres des modèles ont été choisis en raison de la faible incidence de l'outcome d'intérêt au sein de la cohorte (AUPRC) parmi les tests de validation croisée (k-fold=5, nombre de répétitions=10) dans la cohorte RICO d'entrainement (B. Ozenne et al., 2015), la sensibilité, la spécificité, le score F1 et les Valeurs Prédictives Positives (VPP) étant les paramètres d'intérêts.

En résumé, parmi les 5 algorithmes d'apprentissage automatique évalués, les hyper paramètres sélectionnés sont les suivants : Lasso (α=1, λ=0,031), Ridge (α=0, λ=0,556), Elastic net (α=0,35, λ=0,37), PLS (ncomp=1), et le Linear SVM (C=0.367).

L'AUPRC et son intervalle de confiance bootstrap à 95% ont été obtenus avec les packages PRROC (version 1.3.1) et boot (version 1.3-28). Le nombre de rééchantillonnages du boostrap a été défini à N=1000. L'importance des variables relatives dans le modèle Linear SVM a été calculée en utilisant la méthode FIRM issu du package vip (version 0.3.2)(B Greenwell et al., 2018).

L'aire sous la courbe ROC (AUROC), l'intervalle de confiance bootstrap à 95 % et les performances diagnostiques (sensibilité, spécificité, valeurs prédictives positives et négatives et score F1) aux seuils optimaux pour le panel des 9 ARNm ainsi que les paramètres individuels ont été obtenus en tenant compte des valeurs de Youden respectives du progiciel cutpointr (version 1.1.1) défini sur l'ensemble de données d'entraînement, puis appliqués aux valeurs de l'ensemble de données de test. Le score F1 (moyenne harmonique du rappel et de la précision) a été utilisé comme mesure de la précision du modèle en raison du déséquilibre des données.

### 5. Ethique

Le protocole de l'étude RICO a été approuvé par le comité d'éthique (Comité de Protection des Personnes Ile de France 1 - N°IRB/IORG # : IORG0009918) sous le numéro d'accord 2020-A01079-30. Cette étude clinique a été enregistrée sur ClinicalTrials.gov (NCT04392401).

### Résultats

### 1. Caractéristiques cliniques des patients à l'admission en unité de soins intensifs

Les caractéristiques des patients sont présentées dans le tableau 3 ci-dessous. Au total, 309 patients ont été hospitalisés dans 5 hôpitaux de Lyon (FRANCE) entre Août 2020 et Août 2021 et inclus dans la cohorte.

Brièvement et comme précédemment mentionné, 70% des patients étaient des hommes. Les patients ont été admis en unité de soins intensifs avec une médiane de 9 jours après la présentation des premiers symptômes [IQR, 6-11], et présentés notamment un indice de masse corporelle (IMC) médian (kg/m3) de 29,1 [IQR, 26,1-33,2]. En termes de gravité de la maladie, les patients présentaient une PaO2/FiO2 (mmHg) diminuée avec une médiane de 97,5 [IQR : 74,3-146,5], un score SOFA élevé [médiane : 2,0 ; IQR : 1,0-5,0] et un score SAPS II [médiane : 30,0 ; IQR : 23,5-39,0]. De plus, à l'admission, 17,2% des patients ont nécessité une ventilation mécanique invasive.

Tous les patients étaient sous traitement aux corticoïdes avant ou à l'admission (6mg/j de dexaméthasone).

Dans l'ensemble, les patients ont passé à l'hôpital une durée médiane de 18 jours [IQR, 11,0-31,8], dont 8 jours [IQR, 4,0-17,0] en soins intensifs. Un tiers de la cohorte a développé des infections secondaires dont la plupart étaient des pneumopathies (87/99) dont 16 d'origines fongiques.

Enfin, parmi les 309 patients, 52(17%) sont mort à l'issue du 28eme jours post admission.

**[Tableau 3]**

| | **Tous les patients (n = 309)** | **Entrainement** | | | **Test** | | |
|---|---|---|---|---|---|---|---|
| | | **Survivants à 28 jours (n = 179)** | **Non-survivants à 28 jours (n = 37)** | **p value** | **Survivants à 28 jours (n = 78)** | **Non-Survivants à 2 8 jours (n = 15)** | ***p* value** |
| **Démographie** | | | | | | | |
| Age - année | 65.0 [570-720] | 64.0 [55.0-70.0] | 71.0 [690-780] | **<0.001** | 64.5 [55.0-70.0] | 72.0 [68.0-76.0] | **<0.001** |
| Homme - n (%) | 210 (680%) | 119 (66.5%) | 28 (75.7%) | 0.369 | 52 (66.7%) | 11 (73.3%) | 0.767 |
| Indice de masse corporelle (IMC) - kg/m² | 29.1 [26.1-33.2] | 29.1 [25.7-33.1] | 29.6 [27.33-33.0] | 0.674 | 28.7 [26.1-33.4] | 30.1 [28.0-33.2] | 0.335 |
| IMC > 30 - *n* (%) | 128 (43.7%) | 75 (43.9%) | 15 (46.9%) | 0.903 | 30 (40.0%) | 8 (53.3%) | 0.504 |

| **Comorbidités** | | | | | | | |
|---|---|---|---|---|---|---|---|
| Diabètes: aucun - *n (%)* | 213 (69%) | 131 (732%) | 18 (487%) | **0.001** | 55 (70.5%) | 9 (60.0%) | 0.534 |
| Diabètes: avec détérioration - *n* (%) | 15 (4.9%) | 6 (3.4%) | 6 (162%) | | 3 (3.9%) | 0 (0.0%) | |
| Diabètes: sans détérioration organique - *n (%)* | 81 (26.2%) | 42(23.4%) | 13(35.1%) | | 20 (25.6%) | 6 (40.0%) | |
| Score Charlson - points | 1.0 [0.0-2.0] | 1.0 [0.0-1.0] | 2.0 [1.0-4.0] | **< 0.001** | 0.5 [0.0-1.0] | 1.0 [1.0-2.0] | **0.043** |

| **Sévérité clinique à l'admission** | | | | | | | |
|---|---|---|---|---|---|---|---|
| Délai entre les premiers symptômes et l'admission en USI - Jours | 9.0 [6.0-11.0] | 9.0 [7.0-12.0] | 7.5 [5.0-9.8] | **0.013** | 9.0 [6.8-10.3] | 6.0 [5.5-9.0] | **0.041** |
| Score SOFA - points | 2.0 [1.0-5.0] | 2.0 [0.0-5.0] | 4.0 [2.0-6.0] | **0.008** | 2.0 [0.3-3.0] | 3.0 [1.5-7.5] | 0.068 |
| Score SAPS Il - points | 30.0 [23.5-39.0] | 30.0 [23.0-38.8] | 39.0 [33.0-47.0] | **<0.001** | 27.5 [21.0-34.0] | 32.0 [26.8-41.3] | 0.086 |
| PaO₂/FlO₂ - mmHg | 97.5 [74.3-146.5] | 95.0 [77.5-146.0] | 82.0 [70.5-147.8] | 0.376 | 98.0 [89.0-149.0] | 104.5 [93.8-128.3] | 0.844 |
| pH | 7.45 [7.42-7.49] | 7.46 [7.42-7.49] | 7.44 [7.40-7.49] | 0.591 | 7.46 [7.43-7.49] | 7.47 [7.40-7.49] | 0.769 |
| Lactate - mmol/L | 1.65 [1.30-2.00] | 1.70 [1.37-2.02] | 1.90 [1.40-2.20] | 0.326 | 150 [1.30-1.90] | 1.40 [1.30-1.80] | 0.785 |

| **Soutien aux organes** | | | | | | | |
|---|---|---|---|---|---|---|---|
| Ventilation mécanique invasive au jour 0 n (%) | 53 (172%) | 29(16.2%) | 10(27%) | 0.186 | 9 (11.5%) | 5 (33.3%) | **0.046** |
| Médicaments vaso-actifs - *n* (%) | 35 (11.4%) | 19(10.7%) | 8(21.6%) | 0.120 | 6(7.7%) | 2 (13.3%) | 0.611 |
| Traitement de substitution rénale - *n* (%) | 31 (10.0%) | 13 (7.3%) | 11 (29.7%) | **<0.001** | 4 (5.1%) | 3 (20.0%) | 0.080 |

| **Suivi** | | | | | | | |
|---|---|---|---|---|---|---|---|
| MV duration - jours | 14.0 [7.0-27.3] | 17.0 [7.0-34.0] | 12.0 [7.0-20.0] | 0.110 | 22.5 [11.3-30.8] | 12.0 [6.5-15.5] | **0.030** |
| Jours en Unités de Soins Intensifs | 8.0 [4.0-17.0] | 8.0 [3.0-16.0] | 12.0 [8.0-19.0] | **0.017** | 8.0 [5.0-16.8] | 11.0 [5.5-15.5] | 0.871 |
| Jours à l'hôpital | 18.0 [11.0-31.8] | 18.0 [10.0-36.5] | 15.0 [9.0-21.0] | **0.024** | 20.5 [13.0-34.8] | 14.0 [7.5-18.5] | **0.014** |
| Mortalité à 28 jours - *n* (%) | 52 (16.8%) | 0(0%) | 37 (100%) | **<0.001** | 0 (0%) | (100%) | **<0.001** |
| Mortalité à 90 jours - *n* (%) | 66 (21.9%) | 12(6.8%) | 37 (100%) | **<0.001** | 2 (2.7%) | (100%) | **<0.001** |
| Infections acquises en USI - *n* (%) | 99 (33.1 %) | 55(31.6%) | 19(54.3%) | **0.018** | 15 (20.0%) | 10 (66.7%) | **<0.001** |
| Pneumopathies acquises en USI - *n* (% *IAI)* | 87/99 (87.9%) | 48/55 (873%) | 18/19 (94.7%) | 0.366 | 12/15 (800%) | 9/10 (900%) | 0.504 |

| **Paramètres immunologiques à l'admission** | | | | | | | |
|---|---|---|---|---|---|---|---|
| mHLA-DR - AB/C | 8950.0 [6655.5-12173.5] | 9246.0 [6770.0-12827.0] | 7377.5 [4760.8-11413.0] | **0.029** | 8939.0 [6859.8-11038.8 | 8967.0 [7551.5-11118.0] | 0.810 |
| Lymphocytes T CD3 T- nombre absolu | 325.0 [228.0-505.5] | 326.0 [236.5-506.0] | 303.0 [200.0-400.0] | 0.056 | 326.0 [218.0-515.0] | 500.0 [251.0-555.5] | 0.583 |

Les médianes et les écarts interquartiles [Q1-Q3] sont indiqués pour les variables continues où les nombres et pourcentages sont présentés pour les variables catégorielles. Les patients atteints de COVID-19 ont été séparés en deux groupes en fonction de leur statut de survie à 28 jours après l'admission. Les scores de défaillance organique séquentielle (SOFA) et de physiologie aiguë simplifiée II (SAPS II) ont été calculés au cours des 24 premières heures après l'admission. La détresse respiratoire aiguë à l'admission était basée sur la définition de Berlin. Les données ont été comparées à l'aide du test non paramétrique de Mann-Whitney pour les variables continues ou du test exact de Fisher pour les variables catégorielles.

### 2. Association entre l'expression de biomarqueurs et la mortalité des patients à 28 jours post admission en établissement de santé

En utilisant une régression logistique en analyse univariée sur un jeu de donnée de 216 patients, le gène ADGRE3 a été identifié comme significativement associés à la mortalité à 28 jours des patients infectés par virus respiratoire. En particulier, les résultats montrent une sous expression significative de ADGRE3 chez les patients non-survivants par rapport aux patients survivants. Les résultats sont présentés dans le tableau 4 ci-dessous.

**[Tableau 4]**

| | **OR_{IQR} [95% Cl]** | **IQR** | ***p* value** |
|---|---|---|---|
| ADGRE3 | 0.66 [0.45-0.93] | 1.17 | **0.021** |
| mHLA-DR [anticorps/cellules] | 0.97 [0.67-1.30] | 6246 | 0.856 |
| Cellules CD3 T [cellules/pL] | 0.56 [0.31-0.90] | 258.5 | **0.031** |

216 patients ont été inclus dans l'ensemble d'apprentissage, 179 ont survécu jusqu'au 28e jour et 37 sont décédés. L'association entre le statut de survie à 28 jours et le gène ADGRE3 ou les paramètres immunitaires classiques a été réalisée en mettant en oeuvre des modèles de régression logistique univariés. Pour permettre la comparaison entre les modèles, les odds ratios calculés pour ADGRE3 et chaque paramètre immunitaire ont été normalisés à un incrément du premier au troisième quartile (odd ratios inter quartile range, ORIQR). Les valeurs p ≤ 0,05 sont mises en évidence en gras.

Parmi les paramètres cellulaires, la mesure du mHLA-DR n'est pas significativement associés à la mortalité des patients à 28 jours démontrant ainsi tout l'intérêt du biomarqueur ADGRE3.

Le biomarqueur ADGRE3 a ensuite été utilisé dans les 5 modèles d'apprentissage en combinaisons avec un ou plusieurs biomarqueurs additionnels afin de valider des signatures prédictives du risque de décès des patients à 28 jours postadmission.

Les résultats des performances sont présentés dans les tableaux ci-dessous :

### Combinaison ADGRE3 et IL-10

**[Tableau 5]**

| | **Train** | | | | **Test** | | | |
|---|---|---|---|---|---|---|---|---|
| **Modèle** | **AUROC** | **AUROC 95% IC** | **AUPRC** | **AUPRC 95% IC** | **AUROC** | **AUROC 95% IC** | **AUPRC** | **AUPRC 95% IC** |
| Elasticnet | 0,715 | [0.615-0.816] | 0,362 | [0.233-0.525] | 0,717 | [0.573-0.861] | 0,351 | [0.174-0.626] |
| Ridge | 0,713 | [0.62-0.805] | 0,364 | [0.217-0.518] | 0,736 | [0.611-0.861] | 0,323 | [0.156-0.583] |
| Lasso | 0,714 | [0.614-0.815] | 0,358 | [0.227-0.506] | 0,718 | [0.574-0.862] | 0,343 | [0.148-0.633] |
| PLS | 0,98 | [0.963-0.997] | 0,847 | [0.701-0.973] | 0,629 | [0.484-0.775] | 0,241 | [0.122-0.453] |
| svmLin | 0,713 | [0.621-0.805] | 0,364 | [0.234-0.511] | 0,74 | [0.617-0.864] | 0,327 | [0.174-0.615] |

### Combinaison ADGRE3 et CIITA

**[Tableau 6]**

| | **Train** | | | | **Test** | | | |
|---|---|---|---|---|---|---|---|---|
| **Modèle** | **AUROC** | **AUROC 95% IC** | **AUPRC** | **AUPRC 95% IC** | **AUROC** | **AUROC 95% IC** | **AUPRC** | **AUPRC 95% IC** |
| Elasticnet | 0,652 | [0.556-0.748] | 0,32 | [0.198-0.472] | 0,65 | [0.495-0.806] | 0,236 | [0.118-0.433] |
| Ridge | 0,64 | [0.545-0.736] | 0,316 | [0.185-0.476] | 0,654 | [0.5-0.808] | 0,234 | [0.116-0.44] |
| Lasso | 0,64 | [0.544-0.736] | 0,326 | [0.202-0.487] | 0,653 | [0.499-0.807] | 0,233 | [0.122-0.439] |
| PLS | 0,64 | [0.544-0.736] | 0,327 | [0.192-0.475] | 0,652 | [0.498-0.806] | 0,232 | [0.11-0.412] |
| svmLin | 0,637 | [0.541-0.734] | 0,321 | [0.182-0.469] | 0,657 | [0.502-0.813] | 0,24 | [0.121-0.455] |

### Combinaison ADGRE3 et C3AR1

**[Tableau 7]**

| | **Train** | | | | **Test** | | | |
|---|---|---|---|---|---|---|---|---|
| **Modèle** | **AUROC** | **AUROC 95% IC** | **AUPRC** | **AUPRC 95% IC** | **AUROC** | **AUROC 95% IC** | **AUPRC** | **AUPRC 95% IC** |
| Elasticnet | 0,651 | [0.555-0.748] | 0,339 | [0.213-0.493] | 0,61 | [0.478-0.743] | 0,186 | [0.103-0.296] |
| Ridge | 0,648 | [0.548-0.747] | 0,339 | [0.208-0.495] | 0,631 | [0.5-0.762] | 0,195 | [0.109-0.319] |
| Lasso | 0,642 | [0.54-0.743] | 0,336 | [0.189-0.486] | 0,626 | [0.495-0.756] | 0,192 | [0.103-0.3111 |
| PLS | 0,648 | [0.548-0.747] | 0,339 | [0.191-0.485] | 0,631 | [0.5-0.762] | 0,195 | [0.11-0.309] |
| svmLin | 0,648 | [0.55-0.746] | 0,336 | [0.204-0.484] | 0,628 | [0.496-0.76] | 0,194 | [0.114-0.322] |

### Combinaison ADGRE3 et OAS2

**[Tableau 8]**

| | **Train** | | | | **Test** | | | |
|---|---|---|---|---|---|---|---|---|
| **Modèle** | **AUROC** | **AUROC 95% IC** | **AUPRC** | **AUPRC 95% IC** | **AUROC** | **AUROC 95% IC** | **AUPRC** | **AUPRC 95% IC** |
| Elasticnet | 0,645 | [0.528-0.762] | 0,377 | [0.242-0.54] | 0,689 | [0.545-0.832] | 0,259 | [0.134-0.497] |
| Ridge | 0,625 | [0.508-0.741] | 0,354 | [0.212-0.503] | 0,669 | [0.531-0.808] | 0,233 | [0.129-0.423] |
| Lasso | 0,616 | [0.5-0.732] | 0,34 | [0.206-0.474] | 0,669 | [0.535-0.803] | 0,229 | [0.122-0.45] |
| PLS | 0,625 | [0.508-0.742] | 0,355 | [0.217-0.509] | 0,67 | [0.531-0.809] | 0,233 | [0.122-0.455] |
| svmLin | 0,655 | [0.538-0.773] | 0,367 | [0.214-0.512] | 0,7 | [0.553-0.847] | 0,27 | [0.134-0.504] |

### Combinaison ADGRE3, IL-10 et CD74

**[Tableau 9]**

| | **Train** | | | | **Test** | | | |
|---|---|---|---|---|---|---|---|---|
| **Modèle** | **AUROC** | **AUROC 95% IC** | **AUPRC** | **AUPRC 95% IC** | **AUROC** | **AUROC 95% IC** | **AUPRC** | **AUPRC 95% IC** |
| Elasticnet | 0,715 | [0.615-0.816] | 0,362 | [0.233-0.511] | 0,717 | [0.573-0.861] | 0,351 | [0.178-0.673] |
| Ridge | 0,723 | [0.626-0.82] | 0,393 | [0.248-0.561] | 0,759 | [0.629-0.889] | 0,364 | [0.177-0.64] |
| Lasso | 0,716 | [0.615-0.816] | 0,362 | [0.236-0.527] | 0,718 | [0.574-0.862] | 0,354 | [0.174-0.649] |
| PLS | 0,981 | [0.966-0.996] | 0,879 | [0.767-0.978] | 0,726 | [0.586-0.867] | 0,325 | [0.151-0.595] |
| svmLin | 0,717 | [0.619-0.814] | 0,387 | [0.246-0.547] | 0,762 | [0.628-0.897] | 0,416 | [0.2-0.668] |

### Combinaison ADGRE3, C3AR1 et CIITA

**[Tableau 10]**

| | **Train** | | | | **Test** | | | |
|---|---|---|---|---|---|---|---|---|
| **Modèle** | **AUROC** | **AUROC 95% IC** | **AUPRC** | **AUPRC 95% IC** | **AUROC** | **AUROC 95% IC** | **AUPRC** | **AUPRC 95% IC** |
| Elasticnet | 0,674 | [0.582-0.767] | 0,345 | [0.215-0.515] | 0,677 | [0.548-0.806] | 0,222 | [0.12-0.368] |
| Ridge | 0,669 | [0.576-0.762] | 0,343 | [0.202-0.481] | 0,67 | [0.542-0.798] | 0,217 | [0.12-0.389] |
| Lasso | 0,668 | [0.575-0.762] | 0,348 | [0.212-0.508] | 0,676 | [0.544-0.808] | 0,226 | [0.116-0.392] |
| PLS | 0,67 | [0.577-0.763] | 0,343 | [0.203-0.491] | 0,669 | [0.542-0.796] | 0,215 | [0.122-0.374] |
| svmLin | 0,671 | [0.579-0.764] | 0,336 | [0.207-0.493] | 0,658 | [0.538-0.778] | 0,203 | [0.111-0.335] |

### Combinaison ADGRE3, C3AR1, IL-10

**[Tableau 11]**

| | **Train** | | | | **Test** | | | |
|---|---|---|---|---|---|---|---|---|
| **Modèle** | **AUROC** | **AUROC 95% IC** | **AUPRC** | **AUPRC 95% IC** | **AUROC** | **AUROC 95% IC** | **AUPRC** | **AUPRC 95% IC** |
| Elasticnet | 0,715 | [0.615-0.816] | 0,362 | [0.234-0.507] | 0,717 | [0.573-0.861] | 0,351 | [0.16-0.63] |
| Ridge | 0,722 | [0.627-0.817] | 0,395 | [0.229-0.545] | 0,719 | [0.59-0.848] | 0,283 | [0.154-0.517] |
| Lasso | 0,716 | [0.617-0.816] | 0,361 | [0.223-0.51] | 0,726 | [0.583-0.868] | 0,356 | [0.176-0.622] |
| PLS | 0,721 | [0.626-0.817] | 0,407 | [0.25-0.569] | 0,717 | [0.587-0.847] | 0,279 | [0.131-0.487] |
| svmLin | 0,727 | [0.63-0.823] | 0,398 | [0.231-0.563] | 0,724 | [0.593-0.855] | 0,313 | [0.162-0.572] |

### Combinaison ADGRE3, C3AR1 et OAS2

**[Tableau 12]**

| | **Train** | | | | **Test** | | | |
|---|---|---|---|---|---|---|---|---|
| **Modèle** | **AUROC** | **AUROC 95% IC** | **AUPRC** | **AUPRC 95% IC** | **AUROC** | **AUROC 95% IC** | **AUPRC** | **AUPRC 95% IC** |
| Elasticnet | 0,67 | [0.573-0.767] | 0,38 | [0.23-0.5231 | 0,655 | [0.524-0.785] | 0,208 | [0.101-0.34] |
| Ridge | 0,669 | [0.569-0.769] | 0,398 | [0.262-0.551] | 0,665 | [0.531-0.799] | 0,219 | [0.126-0.375] |
| Lasso | 0,663 | [0.564-0.763] | 0,381 | [0.231-0.538] | 0,661 | [0.53-0.7911 | 0,214 | [0.114-0.35] |
| PLS | 0,669 | [0.568-0.769] | 0,397 | [0.246-0.544] | 0,671 | [0.537-0.804] | 0,222 | [0.124-0.378] |
| svmLin | 0,672 | [0.573-0.77] | 0,37 | [0.223-0.531] | 0,675 | [0.542-0.809] | 0,226 | [0.107-0.374] |

### Combinaison ADGRE3, CIITA et IL-10

**[Tableau 13]**

| | **Train** | | | | **Test** | | | |
|---|---|---|---|---|---|---|---|---|
| **Modèle** | **AUROC** | **AUROC 95% IC** | **AUPRC** | **AUPRC 95% IC** | **AUROC** | **AUROC 95% IC** | **AUPRC** | **AUPRC 95% IC** |
| Elasticnet | 0,733 | [0.642-0.823] | 0,351 | [0.219-0.5131 | 0,732 | [0.609-0.854] | 0,308 | [0.159-0.588] |
| Ridge | 0,732 | [0.641-0.824] | 0,373 | [0.234-0.535] | 0,737 | [0.616-0.858] | 0,299 | [0.162-0.558] |
| Lasso | 0,736 | [0.645-0.827] | 0,354 | [0.237-0.519] | 0,735 | [0.611-0.859] | 0,337 | [0.145-0.604] |
| PLS | 0,731 | [0.639-0.822] | 0,372 | [0.233-0.54] | 0,738 | [0.616-0.859] | 0,298 | [0.151-0.54] |
| svmLin | 0,727 | [0.633-0.821] | 0,376 | [0.24-0.529] | 0,744 | [0.619-0.868] | 0,344 | [0.154-0.638] |

### Combinaison ADGRE3, CIITA et OAS2

**[Tableau 14]**

| | **Train** | | | | **Test** | | | |
|---|---|---|---|---|---|---|---|---|
| **Modèle** | **AUROC** | **AUROC 95% IC** | **AUPRC** | **AUPRC 95% IC** | **AUROC** | **AUROC 95% IC** | **AUPRC** | **AUPRC 95% IC** |
| Elasticnet | 0,664 | [0.571-0.757] | 0,323 | [0.195-0.499] | 0,692 | [0.553-0.832] | 0,257 | [0.123-0.463] |
| Ridge | 0,663 | [0.568-0.758] | 0,333 | [0.197-0.494] | 0,692 | [0.551-0.834] | 0,262 | [0.131-0.513] |
| Lasso | 0,657 | [0.563-0.75] | 0,323 | [0.202-0.486] | 0,668 | [0.518-0.818] | 0,244 | [0.141-0.446] |
| PLS | 0,664 | [0.569-0.758] | 0,333 | [0.191-0.484] | 0,696 | [0.556-0.835] | 0,263 | [0.135-0.493] |
| svmLin | 0,671 | [0.568-0.773] | 0,356 | [0.224-0.516] | 0,709 | [0.569-0.849] | 0,274 | [0.15-0.515] |

### Combinaison ADGRE3, OAS2 et IL-10

**[Tableau 15]**

| | **Train** | | | | **Test** | | | |
|---|---|---|---|---|---|---|---|---|
| **Modèle** | **AUROC** | **AUROC 95% IC** | **AUPRC** | **AUPRC 95% IC** | **AUROC** | **AUROC 95% IC** | **AUPRC** | **AUPRC 95% IC** |
| Elasticnet | 0,715 | [0.615-0.816] | 0,362 | [0.244-0.514] | 0,717 | [0.573-0.861] | 0,351 | [0.156-0.645] |
| Ridge | 0,715 | [0.62-0.81] | 0,391 | [0.25-0.55] | 0,744 | [0.618-0.871] | 0,319 | [0.157-0.582] |
| Lasso | 0,715 | [0.615-0.8151 | 0,358 | [0.236-0.513] | 0,72 | [0.577-0.862] | 0,344 | [0.164-0.613] |
| PLS | 0,713 | [0.616-0.81] | 0,395 | [0.254-0.545] | 0,746 | [0.62-0.873] | 0,319 | [0.168-0.594] |
| svmLin | 0,708 | [0.608-0.807] | 0,388 | [0.249-0.549] | 0,752 | [0.623-0.881] | 0,332 | [0.171-0.612] |

### Combinaison ADGRE3, IL-10, CD74 et CIITA

**[Tableau 16]**

| | **Train** | | | | **Test** | | | |
|---|---|---|---|---|---|---|---|---|
| **Modèle** | **AUROC** | **AUROC 95% IC** | **AUPRC** | **AUPRC 95% IC** | **AUROC** | **AUROC 95% IC** | **AUPRC** | **AUPRC 95% IC** |
| Elasticnet | 0,732 | [0.641-0.823] | 0,353 | [0.243-0.514] | 0,732 | [0.611-0.854] | 0,305 | [0.168-0.577] |
| Ridge | 0,725 | [0.632-0.818] | 0,375 | [0.235-0.548] | 0,752 | [0.618-0.886] | 0,328 | [0.172-0.6] |
| Lasso | 0,738 | [0.645-0.83] | 0,379 | [0.241-0.536] | 0,74 | [0.618-0.863] | 0,33 | [0.165-0.616] |
| PLS | 0,994 | [0.988-1] | 0,971 | [0.926-0.997] | 0,659 | [0.506-0.812] | 0,291 | [0.122-0.578] |
| svmLin | 0,712 | [0.619-0.805] | 0,369 | [0.227-0.546] | 0,754 | [0.617-0.891] | 0,335 | [0.18-0.61] |

### Combinaison ADGRE3, C3AR1, CIITA et IL-10

**[Tableau 17]**

| | **Train** | | | | **Test** | | | |
|---|---|---|---|---|---|---|---|---|
| **Modèle** | **AUROC** | **AUROC 95% IC** | **AUPRC** | **AUPRC 95% IC** | **AUROC** | **AUROC 95% IC** | **AUPRC** | **AUPRC 95% IC** |
| Elasticnet | 0,733 | [0.642-0.823] | 0,398 | [0.26-0.57] | 0,738 | [0.623-0.852] | 0,277 | [0.154-0.519] |
| Ridge | 0,735 | [0.644-0.825] | 0,396 | [0.256-0.556] | 0,742 | [0.63-0.854] | 0,279 | [0.157-0.494] |
| Lasso | 0,741 | [0.649-0.832] | 0,383 | [0.243-0.549] | 0,74 | [0.621-0.86] | 0,322 | [0.146-0.583] |
| PLS | 0,731 | [0.641-0.821] | 0,393 | [0.247-0.571] | 0,739 | [0.625-0.853] | 0,277 | [0.14-0.53] |
| svmLin | 0,732 | [0.635-0.828] | 0,397 | [0.242-0.542] | 0,732 | [0.605-0.86] | 0,318 | [0.165-0.593] |

### Combinaison ADGRE3, C3AR1, CIITA et OAS2

**[Tableau 18]**

| | **Train** | | | | **Test** | | | |
|---|---|---|---|---|---|---|---|---|
| **Modèle** | **AUROC** | **AUROC 95% IC** | **AUPRC** | **AUPRC 95% IC** | **AUROC** | **AUROC 95% IC** | **AUPRC** | **AUPRC 95% IC** |
| Elasticnet | 0,686 | [0.595-0.777] | 0,34 | [0.214-0.497] | 0,709 | [0.587-0.832] | 0,241 | [0.126-0.418] |
| Ridge | 0,683 | [0.591-0.775] | 0,362 | [0.228-0.513] | 0,699 | [0.575-0.824] | 0,236 | [0.131-0.384] |
| Lasso | 0,676 | [0.584-0.769] | 0,36 | [0.218-0.516] | 0,697 | [0.569-0.824] | 0,238 | [0.131-0.399] |
| PLS | 0,683 | [0.591-0.776] | 0,369 | [0.218-0.539] | 0,701 | [0.575-0.826] | 0,237 | [0.122-0.419] |
| svmLin | 0,68 | [0.587-0.774] | 0,338 | [0.199-0.508] | 0,703 | [0.58-0.827] | 0,233 | [0.124-0.373] |

### Combinaison ADGRE3, C3AR1, OAS2 et IL-10

**[Tableau 19]**

| | **Train** | | | | **Test** | | | |
|---|---|---|---|---|---|---|---|---|
| **Modèle** | **AUROC** | **AUROC 95% IC** | **AUPRC** | **AUPRC 95% IC** | **AUROC** | **AUROC 95% IC** | **AUPRC** | **AUPRC 95% IC** |
| Elasticnet | 0,728 | [0.631-0.825] | 0,432 | [0.287-0.602] | 0,734 | [0.605-0.8641 | 0,299 | [0.167-0.552] |
| Ridge | 0,726 | [0.631-0.821] | 0,421 | [0.251-0.561] | 0,735 | [0.606-0.864] | 0,297 | [0.149-0.53] |
| Lasso | 0,726 | [0.629-0.824] | 0,4 | [0.253-0.552] | 0,726 | [0.59-0.8631 | 0,337 | [0.15-0.62] |
| PLS | 0,723 | [0.627-0.819] | 0,431 | [0.261-0.594] | 0,73 | [0.602-0.858] | 0,285 | [0.141-0.56] |
| svmLin | 0,727 | [0.63-0.824] | 0,414 | [0.259-0.573] | 0,744 | [0.618-0.871] | 0,318 | [0.172-0.595] |

### Combinaison ADGRE3, OAS2, CIITA et IL-10

**[Tableau 20]**

| | **Train** | | | | **Test** | | | |
|---|---|---|---|---|---|---|---|---|
| **Modèle** | **AUROC** | **AUROC 95% IC** | **AUPRC** | **AUPRC 95% IC** | **AUROC** | **AUROC 95% IC** | **AUPRC** | **AUPRC 95% IC** |
| Elasticnet | 0,727 | [0.633-0.822] | 0,347 | [0.234-0.518] | 0,729 | [0.596-0.862] | 0,36 | [0.149-0.678] |
| Ridge | 0,727 | [0.634-0.82] | 0,387 | [0.239-0.536] | 0,75 | [0.632-0.868] | 0,306 | [0.145-0.567] |
| Lasso | 0,738 | [0.646-0.83] | 0,381 | [0.246-0.557] | 0,743 | [0.623-0.863] | 0,316 | [0.164-0.575] |
| PLS | 0,726 | [0.632-0.819] | 0,389 | [0.242-0.552] | 0,746 | [0.627-0.865] | 0,3 | [0.16-0.587] |
| svmLin | 0,73 | [0.636-0.824] | 0,388 | [0.248-0.551] | 0,751 | [0.629-0.873] | 0,33 | [0.169-0.623] |

### Combinaison ADGRE3, C3AR1, CIITA, IL-10 et OAS2

**[Tableau 21]**

| | **Train** | | | | **Test** | | | |
|---|---|---|---|---|---|---|---|---|
| **Modèle** | **AUROC** | **AUROC 95% IC** | **AUPRC** | **AUPRC 95% IC** | **AUROC** | **AUROC 95% IC** | **AUPRC** | **AUPRC 95% IC** |
| Elasticnet | 0,731 | [0.639-0.823] | 0,386 | [0.232-0.56] | 0,746 | [0.629-0.863] | 0,281 | [0.146-0.5] |
| Ridge | 0,736 | [0.644-0.828] | 0,406 | [0.254-0.578] | 0,747 | [0.631-0.863] | 0,286 | [0.149-0.509] |
| Lasso | 0,737 | [0.644-0.83] | 0,379 | [0.243-0.545] | 0,744 | [0.621-0.868] | 0,347 | [0.173-0.621] |
| PLS | 0,733 | [0.641-0.825] | 0,414 | [0.263-0.573] | 0,746 | [0.63-0.8631 | 0,284 | [0.147-0.513] |
| svmLin | 0,737 | [0.643-0.831] | 0,398 | [0.249-0.564] | 0,75 | [0.627-0.872] | 0,326 | [0.155-0.587] |

### Combinaison ADGRE3, C3AR1, CD177, IL10, CIITA, IL1R2 et OAS2

**[Tableau 22]**

| | **Train** | | | | **Test** | | | |
|---|---|---|---|---|---|---|---|---|
| **Modèle** | **AUROC** | **AUROC 95% IC** | **AUPRC** | **AUPRC 95% IC** | **AUROC** | **AUROC 95% IC** | **AUPRC** | **AUPRC 95% IC** |
| Elasticnet | 0,733 | [0.642-0.824] | 0,349 | [0.223-0.522] | 0,732 | [0.607-0.857] | 0,324 | [0.147-0.59] |
| Ridge | 0,733 | [0.646-0.82] | 0,377 | [0.243-0.535] | 0,738 | [0.612-0.865] | 0,314 | [0.167-0.565] |
| Lasso | 0,717 | [0.617-0.816] | 0,359 | [0.23-0.5211 | 0,721 | [0.577-0.864] | 0,359 | [0.186-0.635] |
| PLS | 0,727 | [0.639-0.815] | 0,381 | [0.238-0.554] | 0,728 | [0.597-0.859] | 0,3 | [0.137-0.553] |
| svmLin | 0,734 | [0.639-0.829] | 0,41 | [0.258-0.556] | 0,732 | [0.603-0.8611 | 0,31 | [0.149-0.581] |

Enfin, les modèles ont été appliqués à une signature contenant le biomarqueur ADGRE3 et les huit biomarqueurs additionnels C3AR1, CD177, IL10, CIITA, IL1R2, OAS2, CD74 et TDRD9 :

**[Tableau 23]**

| **Modèle** | **AUROC_{training} [95% ICI** | **AUPRC_{training} [95% ICI** | **AUROCₜₑₛₜ [95% IC]** | **AUPRCₜₑₛₜ [95% IC]** |
|---|---|---|---|---|
| Elastic Net | 0.715 [0.575-0.844] | 0.361 [0.243-0.524] | 0.721 [0.493-0.938] | 0.380 [0.171-0.662] |
| Ridge | 0.737 [0.612-0.859] | 0.406 [0.257-0.584] | 0.751 [0.575-0.927] | 0.326 [0.164-0.558] |
| Lasso | 0.754 [0.630-0.874] | 0.402 [0.256-0.576] | 0.748 [0.554-0.932] | 0.346 [0.168-0.620] |
| PLS | 0.732 [0.605-0.853] | 0.406 [0.256-0.579] | 0.744 [0.567-0.924] | 0.312 [0.156-0.566] |
| **svmLin** | **0.744 [0.600-0.881]** | **0.431 [0.278-0.610]** | **0.764 [0.536-0.960]** | **0.431 [0.214-0.720]** |

L'ensemble des performances démontre ainsi que la mesure du niveau d'expression de ADGRE3, notamment en combinaison avec la mesure du niveau d'expression d'un ou plusieurs gènes additionnels particuliers permet de déterminer si un patient infecté par un virus respiratoire tel que le SARS-Cov-2, présente un risque accru de décès.

De manière préférée, les combinaisons de biomarqueurs les plus avantageuses les plus pour la prédiction du risque accru de décès chez un sujet infecté par virus respiratoire, sont celles pour lesquelles la majorité des modèles d'apprentissage permettent d'obtenir une AUROC d'au moins 0,7. De plus, la mortalité dans le jeu de donnée test étant de 17%, il est particulièrement avantageux de cibler également les combinaisons pour lesquelles la majorité des modèles permet d'obtenir une AUPRC d'au moins 0,3, de préférence d'au moins 0,32.

### REFERENCES BIBLIOGRAPHIQUES

C. Huang, et al., "Clinical features of patients infected with 2019 novel coronavirus in Wuhan, China" Lancet, 2020, 395, 497, https://doi.org/10.1016/S0140-6736(20)30183-5;
E. Z. Ong, et al.," A Dynamic Immune Response Shapes COVID-19 Progression" Cell Host Microbe, 2020, 27 (6), 879, https://doi.org/10.1016/j.chom.2020.03.021;
F. Venet et al., « Longitudinal assessment of IFN-I activity and immune profile in critically ill COVID-19 patients with acute respiratory distress syndrome"; Crit Care, 2021, 25 (1), 140, https://doi.org/10.1186/s13054-021-03558-w;
Hadjadj, J. et al. "Impaired type I interferon activity and inflammatory responses in severe COVID-19 patients". Science 369, 718-724 (2020**);**
M. S. Abers, et al., "An immune-based biomarker signature is associated with mortality in COVID-19 patients" JCI Insight, 2021, 6 (1):e144455, https://doi.org/10.1172/jci.insight. 144455;
Guardela et al., "50-gene risk profiles in peripheral blood predict COVID-19 outcomes: A rétrospective, multicenter cohort study" EBioMedecine, 2021, https://doi.org/10.1016/j.ebiom.2021.103439;
Y. Levy et al., "CD177, a spécifie marker of neutrophil activation, is associated with coronavirus disease 2019 severity and death" Iscience, 2021, 24(7):102711 DOI: 10.1016/j.isci.2021.102711;
[0169] G. Canedo-Marroquín et al., "SARS-CoV-2: Immune Response Elicited by Infection and Development of Vaccines and Treatments" Front. Immunol, 2020, 11:569760; DOI 10.3389/fimmu.2020.569760;
Chawla, N. V et al.,. SMOTE: synthetic minority over-sampling technique. J. Artif. Int. Res. 16, 321-357 (2002);
Ozenne B. et al, "The precision--recall curve overcame the optimism of the receiver operating characteristic curve in rare diseases". J Clin Epidemiol 68, 855-859, doi:10.1016/j.jclinepi.2015.02.010 (2015);
Greenwell, et al., "A Simple and Effective Model-Based Variable Importance Measure". ArXiv abs/1805.04755 (2018) ;

## Revendications

1. Procédé *in vitro* ou *ex vivo* pour déterminer le risque de décès chez un sujet infecté par un virus respiratoire, ledit procédé comprenant les étapes suivantes de :
a) mesure dans un échantillon biologique dudit sujet du niveau d'expression du gène ADGRE3,
b) comparaison du niveau d'expression du gène ADGRE3 mesuré à l'étape a) avec une valeur de référence prédéterminée.

2. Procédé selon la revendication 1, **caractérisé en ce que** le virus respiratoire est le SARS-CoV-2 ou l'un de ses variants.

3. Procédé selon la revendication 1 ou 2, **caractérisé en ce qu'**il comprend également une étape c) de conclusion quant au risque de décès accru chez ledit sujet sur la base du résultat de la comparaison lorsqu'une sous expression dudit gène au niveau ARNm est mise en évidence.

4. Procédé selon l'une des revendications 1 à 3, **caractérisé en ce que** la valeur de référence correspond à la moyenne du niveau d'expression de ADGRE3 obtenu à partir d'échantillons biologiques issus d'une population de sujets ne présentant aucune infection par un virus respiratoire ou à la moyenne du niveau d'expression de ADGRE3 obtenue à partir d'échantillons biologiques issus d'une population de sujets infectés par un virus respiratoire et dont on sait qu'ils ont survécus après l'infection, notamment dans les 28 jours suivants l'admission en établissement de santé.

5. Procédé selon l'une des revendication 1 à 4, **caractérisé en ce qu'**il comprend en outre une étape de mesure dans l'échantillon biologique dudit sujet de l'expression d'au moins un autre gène additionnel choisi parmi C3AR1, CD177, OAS2, CIITA, IL-10, IL1R2, CD74, TDRD9 et leurs combinaisons.

6. Procédé selon la revendication 5, **caractérisé en ce qu'**il comprend en outre une étape de mesure dans l'échantillon biologique dudit sujet de l'expression d'au moins un autre gène additionnel choisi parmi C3AR1, CD177, OAS2, CIITA, IL-10, IL1R2 et leurs combinaisons, et de préférence parmi C3AR1, CIITA, CD177, IL-10 et leurs combinaisons.

7. Procédé selon l'une des revendication 1 à 6, **caractérisé en ce que** l'expression du(des) gènes est mesurée au niveau ARNm.

8. Procédé selon l'une des revendications 1 à 7, **caractérisé en ce que** l'échantillon biologique est un échantillon de sang, de préférence un échantillon de sang total.

9. Procédé selon l'une des revendications 1 à 8, **caractérisé en ce que** l'expression est mesurée par amplification, séquençage ou hybridation.

10. Procédé selon l'une des revendications 1 à 9, **caractérisé en ce que** l'expression est mesurée par amplification via une RT-PCR, de préférence une RT-qPCR.

11. Procédé selon l'une des revendications 1 à 10, **caractérisé en ce que** l'expression est normalisée par rapport à l'expression d'un ou plusieurs gènes de ménage.

12. Kit pour la mesure *in vitro* ou *ex vivo* de l'expression de ADGRE3 dans un échantillon biologique comprenant des moyens de détermination du niveau d'expression de ADGRE3 dans ledit échantillon, lesdits moyens étant choisis parmi des amorces d'amplification ou des sondes.

13. Kit selon la revendication 12, **caractérisé en ce qu'**il comprend un échantillon contrôle positif calibré pour contenir la quantité de ADGRE3 qui correspond à la quantité ou la concentration représentative du niveau d'expression mesuré au niveau ARNm dans un pool d'échantillons de sujets ne présentant pas d'infection par un virus respiratoire, et/ou un échantillon contrôle négatif calibré pour contenir la quantité de ADGRE3 qui correspond à la quantité moyenne mesurée dans un pool d'échantillons de sujets qui n'ont pas survécu suite à une infection par un virus respiratoire.

14. Kit selon la revendication 12 ou 13, **caractérisé en ce qu'**il comprend des moyens de détermination du niveau d'expression d'au moins un autre gène additionnel sélectionné parmi C3AR1, CD177, OAS2, CIITA, IL-10, IL1R2, CD74, TDRD9 et leurs combinaisons, de préférence sélectionné parmi C3AR1, CD177, CIITA, IL-10, IL1R2 et leurs combinaisons, et de préférence encore sélectionné parmi C3AR1, CIITA, IL-10 et leurs combinaisons.

15. Utilisation du kit selon l'une des revendications 12 à 14 pour la détermination du risque de décès, de préférence du risque de décès dans les 28 jours post-admission en établissement de santé, d'un sujet infecté par un virus respiratoire tel que le SARS-CoV-2 ou l'un de ses variants.
